# EUROPÄISCHE PATENTSCHRIFT

(11) **EP 0 859 798 B1**
(45) Veröffentlichungstag und Bekanntmachung des Hinweises auf die Patenterteilung: **31.01.2001**
(21) Anmeldenummer: 97918969.3
(22) Anmeldetag: 07.08.1997
(51) Int. Cl.: C08F 4/60, C08F 10/00, C07F 17/00

(54) **KATALYSATORSYSTEM**
CATALYST SYSTEM
SYSTEME DE CATALYSEUR

(30) Priorität: 16.08.1996 DE 19632919
(43) Veröffentlichungstag der Anmeldung: 26.08.1998
(73) Patentinhaber: TARGOR GmbH, 55116 Mainz (DE)
(72) Erfinder: ERKER, Gerhard, D-48159 Münster (DE); BERTULEIT, Axel, D-23560 Lübeck (DE); RIEDEL, Michael, D-45130 Essen (DE); BOSCH, Boris, D-48143 Münster (DE)
(74) Vertreter: Stark, Vera, Dr.
(86) Internationale Anmeldenummer: EP9704310
(87) Internationale Veröffentlichungsnummer: WO9807760

(56) Entgegenhaltungen:
- EP-A- 0 255 296
- DELGADO E ET AL: "Thiolate derivatives of bis(diphenylphosphinocyclopentadienyl)tita nium Ti-Pt and Ti-Pd heterobimetallic compounds" JOURNAL OF ORGANOMETALLIC CHEMISTRY, Bd. 494, Nr. 1, 31.Mai 1995, Seite 261-265 XP004023982 in der Anmeldung erwähnt
- MAKOTO MITANI ET AL: "LIGAND EFFECTS ON OLEFIN POLYMERIZATIONS WITH MULTINUCLEAR ZIRCONOCENE CATALYSTS HAVING DIMETHYLSILYLFERROCENE AS A CYCLOPENTADIENYL SUBSTIUENT" MACROMOLECULAR CHEMISTRY AND PHYSICS, Bd. 197, Nr. 6, Juni 1996, Seiten 1815-1822, XP000634256
- LINDENBERG: "Dinuclear phosphido- and arsenido-bridged early/late transition metal complexes." JOURNAL OF ORGANOMETALLIC CHEMISTRY, Bd. 515, 1996, Seiten 19-25, XP004035985

## Beschreibung

Die vorliegende Erfindung betrifft ein Katalysatorsystem enthaltend eine bimetallische Übergangsmetallverbindung und einen Cokatalysator sowie ein Verfahren zur Herstellung der bimetallischen Übergangsmetallverbindung sowie der Verwendung der bimetallischen Übergangsmetallverbindung als Katalysatorkomponente, insbesondere bei der Herstellung von Polyolefinen sowie bei der Hydroformylierung von olefinisch ungesättigten Verbindungen.

Aus der Literatur ist die Herstellung von Polyolefinen mit löslichen Metallocenen sowie Monocyclopentadienylverbindungen in Kombination mit Aluminoxanen oder anderen Cokatalysatoren, die aufgrund ihrer Lewis-Acidität die neutrale Übergangsmetallverbindung in ein Kation überführen und stabilisieren können, bekannt (EP-A 129 368, EP-A 351 392, EP-A 416 815).

Metallocene und Monocyclopentadienylverbindungen sind nicht nur hinsichtlich der Polymerisation oder Oligomerisation von Olefinen von großem Interesse. Sie können auch als Hydrier-, Epoxidations-, Isomerisierungs- und C-C-Kupplungskatalysatoren eingesetzt werden (Chem. Rev. 1992, 92, 965-994).

Bei Einsatz löslicher Metallocenverbindungen auf der Basis von Bis(cyclopentadienyl)zirkon-dialkyl bzw. dihalogenid in Kombination mit oligomeren Aluminoxanen erhält man ataktische Polymere, die wegen ihrer unausgewogenen und ungenügenden Produkteigenschaften technisch nur von geringer Bedeutung sind. Außerdem sind bestimmte Olefincopolymere nicht zugänglich.

Derivate des Zirkonocendichlorids, in denen die beiden substituierten Cyclopentadienylgruppen über eine Methyl-, Ethylen- oder eine Dimethylsilylbrücke miteinander verbunden sind, können aufgrund ihrer konformativen Starrheit als Katalysatoren zur isospezifischen Polymerisation von Olefinen eingesetzt werden (EP-A 316 155).

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise solche der Gruppe Vlllb des Periodensystems der Elemente, katalysiert. Bei den in der Technik eingeführten Verfahren wird zum Beispiel ein Rhodium-Katalysator in Form modifizierter Hydridorhodiumcarbonyle eingesetzt, die zusätzliche Liganden, inbesondere tertiäre, organische Phosphane oder Phosphite erhalten. Meist liegen die Liganden im Überschuß vor, so daß das Katalysatorsystem aus Komplexverbindung und Ligand besteht.

Es ist bekannt, daß Diazadienstabilisierte Komplexe der Gruppe Vlllb des Periodensystems, bevorzugt Nickel- und Palladiumverbindungen, die Homo- und Copolymerisation von Olefinen und polaren Monomeren wie Methacrylat katalysieren (J. Am. Chem. Soc, 1996, 118, 267; J. Am. Chem. Soc. 1995m 117, 6414).

Die vorliegende Erfindung betrifft somit ein Katalysatorsystem bestehend aus einer bimetallischen Übergangsmetallverbindung und einem Cokatalysator sowie die Verwendung des Katalysatorsystems in der Homo- und Copolymerisation von Olefinen und polaren Monomeren, sowie in der Verwendung der bimetallischen Übergangsmetallverbindung in der Hydroformylierung von Olefinen. Weiterhin ist auch eine Kombination dieser beiden Katalyseprozesse denkbar.

Die vorliegende Erfindung betrifft somit ein Katalysatorsystem enthaltend a) mindestens einem Cokatalysator und b) mindestens eine bimetallische Übergangsmetallverbindung der Formel I 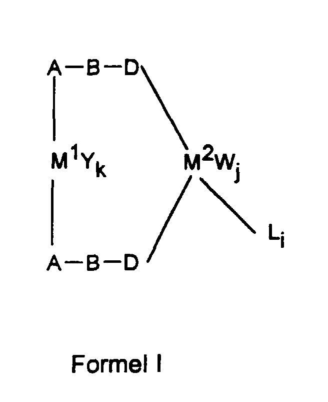 worin
M¹ ein Übergangsmetall der Gruppe III b, IV b Vb, oder VI b des Periodensystems der Elemente ist,
Y gleich oder verschieden sind und ein Wasserstoffatom, eine C₁-C₄₀-kohlenwasserstoffhaltige Gruppe wie eine C₁-C₂₀-Alkyl, eine C₁-C₂₀-Alkoxy-, eine C₆-C₂₀-Aryl, eine C₂-C₂₀-Alkenyl-, eine C₇-C₄₀-Alkylaryl oder eine C₈-C₄₀-Arylalkenylgruppe, eine OH-Gruppe, ein Halogenatom, eine NR¹₂-Gruppe, worin R¹ ein Halogenatom, eine C₁-C₁₀ Alkylgruppe oder eine C₆-C₁₀-Arylgruppe ist, bedeuten oder
Y eine substituierte oder unsubstituierte Butadieneinheit sind,
k eine ganze Zahl ist, die der Wertigkeit des Übergangsmetallatoms M¹ minus zwei entspricht und im Fall, wenn Y gleich Butadien bedeutet gleich 1 ist,
A gleich oder verschieden ein substituierter oder unsubstituierter Cyclopentadienylligand sind, wobei mindestens einer substituiert sein muß,
B gleich oder verschieden sind und 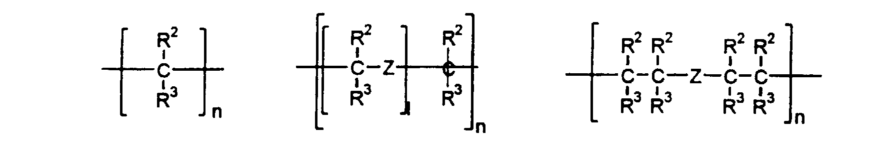 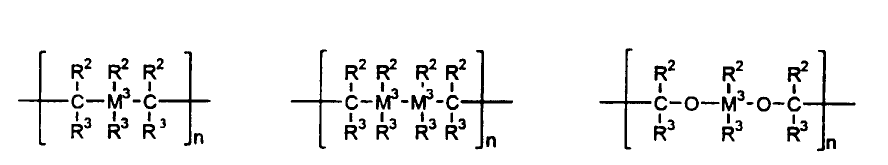 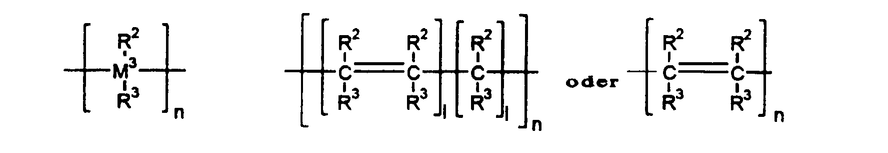 bedeuten, wobei n eine ganze Zahl von 0 bis 20 ist, l eine ganze Zahl von 1 bis 20 ist, Z gleich

   〉NR⁴, 〉CO, 〉PR⁴, 〉P(O)R⁴, 〉SO,

   SO₂, O oder S ist, worin R⁴ ein Halogenatom, eine C₁-C₁₀ Alkylgruppe oder eine C₆-C₁₀-Arylgruppe ist,
R² und R³ gleich oder verschieden sind und ein Wasserstoffatom, ein Halogenatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, oder jeweils zwei Reste R², jeweils zwei Reste R³, oder je ein Rest R² und R³ jeweils mit den sie verbindenen Atomen einen oder mehrere Ringe bilden und M³ Silizium, Germanium oder Zinn ist,
D gleich oder verschieden sind und

   X-R⁵ ₘ

   bedeuten und X an B kovalent und an M² koordinativ gebunden sind, wobei X gleich ein Element der Gruppe Va oder Vla des Periodensystems der Elemente bedeutet und R⁵ gleich oder verschieden ein Wasserstoffatom, ein Halogenatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, oder jeweils zwei Reste R⁵ mit den sie verbindenen Atomen einen oder mehrer Ringe bilden und m gleich 1 oder 2 ist,
M² ein Metall der Gruppe VI b, VII b, VIII b, IX b oder X b des Periodensystems der Elemente ist,
W gleich oder verschieden sind und ein Wasserstoffatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe, ein Halogenatom, eine OR¹-Gruppe, eine NR¹₂-Gruppe, worin R¹ ein Wasserstoffatom, ein Halogenatom, eine C₁-C₁₀ Alkylgruppe oder eine C₆-C₁₀-Arylgruppe ist oder eine substituierte oder unsubstituierte Butadieneinheit ist, bedeuten,
j eine ganze Zahl von 0 bis 6 ist, die der Wertigkeit des Übergangsmetallatoms M² entspricht
L einen neutralen Donorliganden bedeutet wie Kohlenmonoxid, ein tertiäres Phoshpan der allgemeinen Formel PR₃⁶, worin R⁶ gleich oder verschieden sind und ein Wasserstoffatom, ein Halogenatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, oder jeweils zwei Reste R⁶ mit den sie verbindenen Atomen einen oder mehrer Ringe bilden, ein tertiäres Phosphit der allgemeinen Formel P(O)R₃⁶, worin R⁶ gleich oder verschieden sind und ein Halogenatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, oder jeweils zwei Reste R⁶ mit den sie verbindenen Atomen einen oder mehrer Ringe bilden, ein Nitril der allgemeinen Formel R⁶-CN, worin R⁶ eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe ist, oder ein Isonitril der allgemeinen Formel R⁶-NC worin R⁶ eine C₁-C₄₀-kohlenwasserstoflhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, und/oder weitere in der Literatur beschriebene neutrale Donorliganden, und
i eine ganze Zahl von 0 bis 10 ist.

Die beiden Cyclopentadienyl liganden A können unverbrückt oder verbrückt sein. Beispiele für Verbrückungen zwischen den Cyclopentadienylliganden A sind: Dimethylsilandiyl, Methylphenylsilandiyl, Diphenylsilandiyl, Dimethylgermandiyl, 1,2-Tetramethyldisilandiyl, 1,2-Ethyliden, 1,2-Propyliden, 2,2-Propyliden, 1,2-Butyliden, 1,3-Propyliden und 1,4-Butyliden.

Eine bevorzugte Ausführungsform des erfindungsgemäßen Katalysatorsystems enthält einen Cokatalysator und eine bimetallische Übergangsmetallverbindung der Formel II und 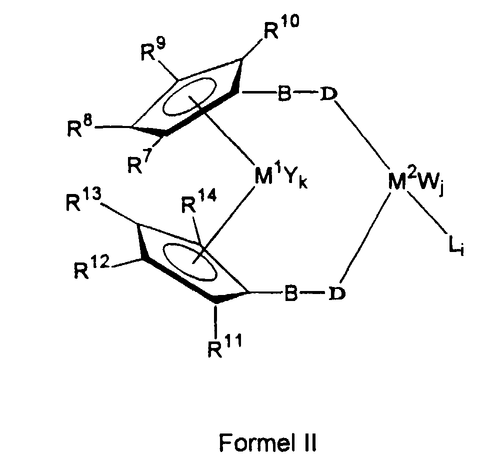 worin
M¹ ein Übergangsmetall der Gruppe III b, IV b, V b oder VI b des Periodensystems der Elemente ist,
Y bevorzugt gleich oder verschieden sind und ein Wasserstoffatom, eine C₁-C₂₀-kohlenwasserstoffhaltige Gruppe wie eine C₁-C₁₀-Alkyl-, eine C₁-C₁₀-Alkoxy-, eine C₆-C₁₀-Aryl-, eine C₂-C₁₀-Alkenyl-, eine C₇-C₂₀-Alkylaryl- oder eine C₈-C₂₀-Arylalkenylgruppe, ein Halogenatom, eine NR¹₂-Gruppe, worin R¹ ein Halogenatom, eine C₁-C₁₀ Alkylgruppe oder eine C₆-C₁₀-Arylgruppe ist,
k eine ganze Zahl ist, die der Wertigkeit des Übergangsmetallatoms M¹ minus zwei entspricht,
R⁷, R⁸, R⁹, R¹⁰, R¹¹, R¹², R¹³ und R¹⁴ gleich oder verschieden sind und ein Wasserstoffatom, ein Halogenatom, eine C₁-C₄₀-kohlenwasserstoffhaltige Gruppe wie eine C₁-C₂₀-Alkyl-, eine C₁-C₁₀-Alkoxy-, eine C₆-C₂₀-Aryl-, eine C₂-C₁₀-Alkenyl-, eine C₇-C₄₀-Alkylaryl- oder eine C₈-C₄₀-Arylalkenylgruppe, einen -SiR¹₃,-NR¹₂, -SiOR¹₃, -SiSR¹₃ oder -PR¹₂-Rest bedeuten, worin R¹ ein Halogenatom, eine C₁-C₁₀ Alkylgruppe oder eine C₆-C₁₀-Arylgruppe ist, oder zwei oder mehr benachbarte Reste R⁷, R⁸, R⁹ und R¹⁰ oder R¹¹, R¹², R¹³ und R¹⁴ zusammen mit den sie verbindenen Atomen ein Ringsystem bilden, welches bevorzugt 4 bis 40 besonders bevorzugt 6 bis 15 Kohlenstoffatome enthält, oder ein Rest R⁷, R⁸, R⁹ oder R¹⁰ mit einem Rest R¹¹, R¹², R¹³ oder R¹⁴ eine Verbrückung zwischen den beiden Cyclopentadienylringen darstellt,
B gleich 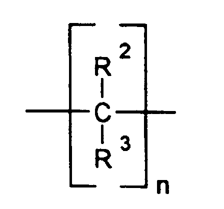 ist, wobei n eine ganze Zahl von 1 bis 8 insbesondere 1,2,3 oder 4 ist und R² und R³ gleich oder verschieden sind und ein Wasserstoffatom, ein Halogenatom, eine C₁-C₂₀-kohlenwasserstoffhaltige Gruppe wie eine C₁-C₁₀-Alkyl-, eine C₁-C₁₀-Alkoxy-, eine C₆-C₁₀-Aryl-, eine C₂-C₁₀-Alkenyl-, eine C₇-C₂₀-Alkylaryl- oder eine C₈-C₂₀-Arylalkenylgruppe bedeuten, oder jeweils zwei Reste R², jeweils zwei Reste R³, oder R² und R³ jeweils mit den sie verbindenen Atomen einen oder mehrere Ringe bilden,
D gleich

   X-R⁵ ₘ

   bedeutet, wobei X gleich ein Element der Gruppe Va des Periodensystems der Elemente, wie Stickstoff oder Phosphor, oder der Gruppe VI a des Periodensystems der Elemente, wie Sauerstoff oder Schwefel, bedeutet und R⁵ gleich oder verschieden ein Wasserstoffatom oder eine C₁-C₁₀-kohlenwasserstoffhaltige Gruppe wie eine C₁-C₁₀-Alkyl-, eine C₁-C₁₀-Alkoxy-, eine C₆-C₁₀-Aryl-, eine C₂-C₁₀-Alkenyl-, eine C₇-C₁₀-Alkylaryl- oder eine C₈-C₁₀-Arylalkenylgruppe bedeuten, oder jeweils zwei Reste R⁵ mit den sie verbindenen Atomen einen oder mehrere Ringe bilden, sowie im Fall, wenn X gleich Stickstoff oder Phosphor bedeutet m gleich 2 ist, und im Fall, wenn X gleich Sauerstoff oder Schwefel ist m gleich 1 ist, M² bevorzugt ein Metall der Gruppe VIII b des Periodensystems der Elemente wie Eisen, Ruthenium, Osmium, Cobalt, Rhodium, Iridium, Nickel, Palladium oder Platin ist,
W bevorzugt gleich oder verschieden sind und ein Wasserstoffatom, eine C₁-C₂₀-kohlenwasserstoffhaltige Gruppe wie eine C₁-C₁₀-Alkyl-, eine C₁-C₁₀-Alkoxy-, eine C₆-C₁₀-Aryl-, eine C₂-C₁₀-Alkenyl-, eine C₇-C₁₀-Alkylaryl- oder eine C₈-C₁₀-Arylalkenylgruppe, ein Halogenatom, eine NR¹₂-Gruppe, worin R¹ ein Halogenatom, eine C₁-C₁₀ Alkylgruppe oder eine C₆-C₁₀-Arylgruppe ist j eine ganze Zahl ist, die der Wertigkeit des Übergangsmetallatoms M² entspricht, L einen neutralen Donorliganden bedeutet wie Kohlenmonoxid, ein tertiäres Phoshpan der allgemeinen Formel PR₃⁶, worin R⁶ gleich oder verschieden sind und eine C₁-C₂₀-kohlenwasserstoffhaltige Gruppe wie eine C₁-C₁₀-Alkyl-, eine C₁-C₁₀-Alkoxy-, eine C₆-C₂₀-Aryl-, eine C₂-C₁₀-Alkenyl-, eine C₇-C₂₀-Alkylaryl- oder eine C₈-C₂₀-Arylalkenylgruppe bedeuten, oder jeweils zwei Reste R⁶ mit den sie verbindenen Atomen einen Ring bilden, ein tertiäres Phosphit der allgemeinen Formel P(O)R₃⁶, worin R⁶ gleich oder verschieden sind und eine C₁-C₂₀-kohlenwasserstoffhaltige Gruppe wie eine C₁-C₁₀-Alkyl-, eine C₁-C₁₀-Alkoxy-, eine C₆-C₂₀-Aryl-, eine C₂-C₁₀-Alkenyl-, eine C₇-C₂₀-Alkylaryl- oder eine C₈-C₂₀-Arylalkenylgruppe bedeuten, oder jeweils zwei Reste R⁶ mit den sie verbindenen Atomen einen Ringe bilden, ein Nitril der allgemeinen Formel R⁶-CN, worin R⁶ eine C₁-C₂₀-kohlenwasserstoffhaltige Gruppe wie eine C₁-C₁₀-Alkyl-, eine C₁-C₁₀-Alkoxy-, eine C₆-C₂₀-Aryl-, eine C₂-C₁₀-Alkenyl-, eine C₇-C₂₀-Alkylaryl- oder eine C₈-C₂₀-Arylalkenylgruppe ist, oder ein Isonitril der allgemeinen Formel R⁶-NC worin R⁶ eine C₁-C₂₀-kohlenwasserstoffhaltige Gruppe wie eine C₁-C₁₀-Alkyl-, eine C₁-C₁₀-Alkoxy-, eine C₆-C₂₀-Aryl-, eine C₂-C₁₀-Alkenyl-, eine C₇-C₂₀-Alkylaryl- oder eine C₈-C₂₀-Arylalkenylgruppe bedeuten und i 1,2,3,4,5 oder 6 ist.

Beispiele für Verbrückungen zwischen den Cyclopentadienylliganden sind: Dimethylsilandiyl, Methylphenylsilandiyl, Diphenylsilandiyl, Dimethylgermandiyl, 1,2-Tetramethyldisilandiyl, 1,2-Ethyliden, 1,2-Propyliden, 2,2-Propyliden, 1,2-Butyliden, 1,3-Propyliden und 1,4-Butyliden.

Besonders bevorzugt sind bimetallische Übergangsmetallverbindungen der Formel II als Katalysatorkomponente,
worin
M¹ ein Übergangsmetall der Gruppe IV b des Periodensystems der Elemente, insbesondere Titanium, Zirkonium oder Hafnium ist,
Y bevorzugt gleich oder verschieden sind und eine C₁-C₁₀-kohlenwasserstoffhaltige Gruppe wie eine C₁-C₄-Alkyl, insbesondere Methyl, Ethyl, Isopropyl, Butyl, oder eine C₆-C₁₀-Aryl, inbesondere Phenyl, oder ein Halogenatom, insbesondere Chlor, Brom, Fluor ist und und k eine ganze Zahl ist, die der Wertigkeit des Übergangsmetallatoms M¹ minus zwei entspricht,
R⁷, R⁸, R⁹, R¹⁰, R¹¹, R¹², R¹³ und R¹⁴ gleich oder verschieden sind und ein Wasserstoffatom, eine C₁-C₁₀-kohlenwasserstoffhaltige Gruppe wie eine C₁-C₁₀-Alkyl, insbesondere eine Methyl, Ethyl, Isopropyl, Tert.Butyl, eine C₆-C₁₀-Aryl, insbesondere Phenyl, eine C₇-C₁₀-Alkylaryl, insbesondere Benzyl oder zwei oder mehr benachbarte Reste R⁷, R⁸, R⁹ oder R¹⁰ oder R¹¹, R¹², R¹³ oder R¹⁴ zusammen mit den sie verbindenen Atomen ein Ringsystem bilden, welches bevorzugt 4 bis 40 besonders bevorzugt 6 bis 15 Kohlenstoffatome enthält,
B gleich 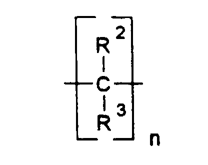
ist, wobei n eine ganze Zahl 1,2,3 oder 4 ist und R² und R³ gleich sind und ein Wasserstoffatom, eine C₁-C₁₀-kohlenwasserstoffhaltige Gruppe wie C₁-C₁₀-Alkyl, insbesondere Methyl, Ethyl, Butyl oder C₆-C₁₀-Aryl, insbesondere Phenyl, bedeutet oder je ein Rest R² und R³ jeweils mit den sie verbindenen Atomen einen oder mehrer Ringe bilden,
D gleich

   X-R⁵ ₂

   bedeutet, wobei X gleich ein Element der Gruppe Va des Periodensystems der Elemente, inbesondere Stickstoff oder Phosphor, bedeutet und R⁵ gleich oder verschieden ist und ein Wasserstoffatom, eine C₁-C₁₀-kohlenwasserstoffhaltige Gruppe wie C₁-C₁₀-Alkyl, inbesondere Methyl, Ethyl, lsoproyl, Butyl oder C₆-C₁₀-Aryl, insbesondere Phenyl bedeutet oder jeweils zwei Reste R⁵ mit den sie verbindenen Atomen einen oder mehrere Ringe bilden,
M² bevorzugt ein Metall der Gruppe VIII b des Periodensystems der Elemente, insbesondere bevorzugt Ruthenium, Cobalt, Rhodium, Nickel oder Palladium ist, W bevorzugt gleich sind und ein Wasserstoffatom, eine C₁-C₁₀-kohlenwasserstoffhaltige Gruppe wie eine C₁-C₁₀-Alkyl, insbesondere Methyl, Ethyl, Isopropyl, Butyl oder eine C₆-C₁₀-Aryl, insbesondere Phenyl, oder ein Halogenatom, insbesondere Brom, Chlor, Fluor oder eine NR¹₂-Gruppe, worin R¹ ein Halogenatom, eine C₁-C₁₀ Alkylgruppe oder eine C₆-C₁₀-Arylgruppe ist und j eine ganze Zahl ist, die der Wertigkeit des Übergangsmetallatoms M² entspricht, L einen neutralen Donorliganden wie Kohlenmonoxid, ein tertiäres Phoshpan der allgemeinen Formel PR₃⁶, worin R⁶ gleich sind und eine C₁-C₁₀-kohlenwasserstoffhaltige Gruppe wie eine C₁-C₁₀-Alkyl, insbesondere Methyl, Ethyl, Isopropyl, Butyl, Hexyl, eine C₆-C₁₀-Aryl, insbesondere Phenyl, Tolyl, Methoxyphenyl oder eine C₇-C₁₀-Alkylaryl, insbesondere Benzyl, bedeuten, ein tertiäres Phosphit der allgemeinen Formel P(O)R₃⁶, worin R⁶ gleich sind und eine C₁-C₁₀-kohlenwasserstoffhaltige Gruppe wie eine C₁-C₁₀-Alkyl, insbesondere Methyl, Ethyl, Isopropyl, Butyl, Hexyl, eine C₆-C₁₀-Aryl, insbesondere Phenyl, Tolyl, Methoxyphenyl oder eine C₇-C₁₀-Alkylaryl, insbesondere Benzyl, bedeuten, ein Nitril der allgemeinen Formel R⁶-CN, worin R⁶ eine C₁-C₁₀-kohlenwasserstoffhaltige Gruppe wie eine C₁-C₁₀-Alkyl, insbesondere Methyl, Ethyl, Isopropyl, Butyl, Hexyl, eine C₆-C₁₀-Aryl, insbesondere Phenyl, Tolyl, Methoxyphenyl oder eine C₇-C₁₀-Alkylaryl, insbesondere Benzyl, bedeuten, oder ein Isonitril der allgemeinen Formel R⁶-NC worin R⁶ eine C₁-C₂₀-kohlenwasserstoffhaltige Gruppe wie eine C₁-C₁₀-Alkyl, insbesondere Methyl, Ethyl, Isopropyl, Butyl, Hexyl, eine C₆-C₁₀-Aryl, insbesondere Phenyl, Tolyl, Methoxyphenyl oder eine C₇-C₁₀-Alkylaryl, insbesondere Benzyl bedeuten, und i 1,2,3 oder 4 ist.

Anhand der folgenden Verbindungen soll die Nomenklatur erläutert werden:
trans-Dichlorobis{[1-(1-methyl-1-diphenylphosphinoethyl)-η⁵-cyclopentadienyl]dichlorozirconium(IV)-κ²P,P'}palladium(II) 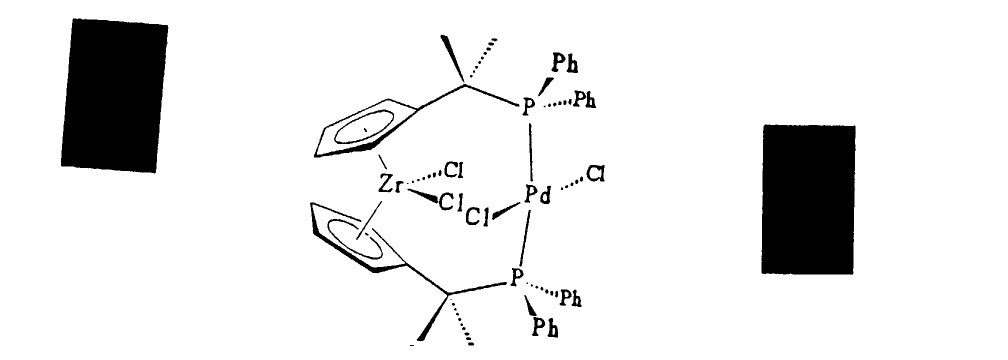
Di-µ-chlorobis[bis(diphenylphosphino-η⁵-cyclopentadienyl)dichlorozirconium(lV)-K²P,P']-rhodium(I)

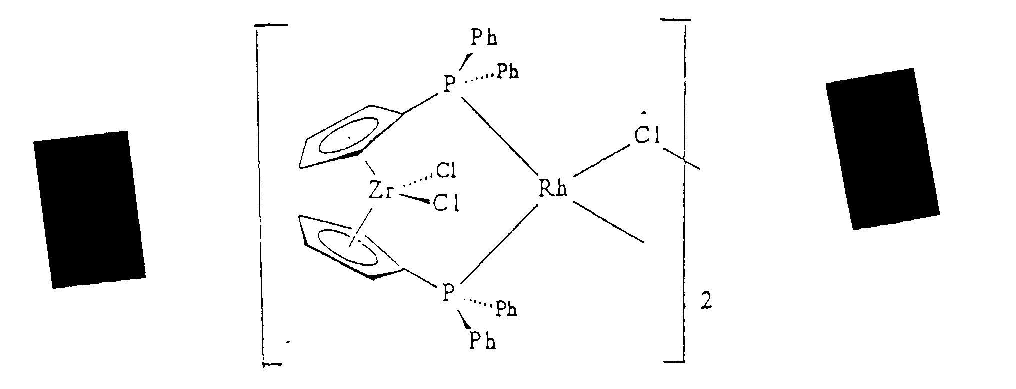

Beispiele für erfindungsgemäße Übergangsmetallverbindungen sind:
trans-Dichlorobis{[1-(1-methyl-1 -diphenylphosphinoethyl)-η⁵-cyclopentadienyl]dichlorozirconium(lV)-κ²P, P'}palladium(II) Dichlorobis{[1-(1-methyl-1-diphenylphosphinoethyl)-η⁵-cyclopentadienyl]dichlorozirconium(lV)-κ²P,P'}platin(ll) µ-{Bis[1-(methyl-1-diphenylphosphinoethyl)-η⁵-cyclopentadienyl]dichlorozirconium(IV)-κP:κP'}bis[(chlorocyclooctadien)rhodium(I)] Dichlorobis{[1-(1-methyl-1-di-p-tolylphosphinoethyl)-η⁵-cyclopentadienyl]dichlorozirconium(IV)-κ²P,P'}palladium(II) Dichlorobis{[1-(1-methyl-1-ditolylphosphinoethyl)-η⁵-cyclopentadienyl]dichlorozirconium(lV)-κ²P,P'}platin(II) Pentachlorotetrahydrofuranopalladium(IV)-chlorobis{[1-(1-tert.butyldiphenylphosphinomethyl)-η⁵-cyclopentadienyl]chlorozirconium(lV)-κ²P,P']}µ-chloropalladat(ll)
Pentachlorotetrahydrofuranopalladium(lV)-chlorobis{[1-(1-tert.butyldiphenylphosphinomethyl)-η⁵-cyclopentadienyl]chlorozirconium(lV)-κ²P,P']}µ-chloropalladat(ll)
   µ-Bis[1-phenyldiphenylphosphinomethyl)-η⁵-cyclopentadienyl]dichlorozirconium(IV)-κP:κP'}bis[(chlorocyclooctadien)rhodium(I)]
cis-Dichloro)bis[η⁵-(1-dimethylaminoethyl)cyclopentadienyl]dichlorotitan-κ²P,P']-palladium(II)
   Dichlorobis[η⁵-(1-dimethylaminoethyl)cyclopentadienyl]dichlorozirconium-(lV)-κ²P,P']-platin(II)
   Di-µ-chlorobis[bis[η⁵-(1-dimethylaminobenzyl)cyclopentadienyl]dichloro-zirconium]-κ²P,P']-rhodium(I)
   Carbonylchlorobis[η⁵-(1-dimethylamino-1-methylethyl)cyclopentadienyl]-chloro-µ-chlorotitanium(lV)-κ²P,P']rhodium(I)
   Dichlorobis[η⁵-(1-dimethylaminoethyl)cyclopentadienyl]dichlorozirconium-(lV)-κ²P,P']-nickel(II)
   [η⁵-(1-Dimethylamino-1-methylbenzyl)cyclopentadienyl][η⁵-cyclopentadienyl]dichlorozirconium
cis-Dichlorobis[(diphenylphosphino-η⁵-cyclopentadienyl)dichlorozirconium-(lV)-κ²P,P']-palladium(II)
   Dichlorobis[(diphenylphosphino-η⁵-cyclopentadienyl)dichlorozirconium(lV)-κ²P,P']-platin(ll)
   Dichlorobis[(diphenylphosphino-η⁵-cyclopentadienyl)dichlorozirconium(lV)-κ²P,P']-platin(ll)
   Carbonylchloro[bis(diphenylphosphino-η⁵-cyclopentadienyl )chloro-µ-chlorozirconium(lV)-κ²P,P']rhodium(I)
   Carbonylhydrido[bis(diphenylphosphino-η⁵-cyclopentadienyl)dichloro-zirconium(IV)-κ²P,P']triphenylphosphinorhodium(I)
   Di-µ-chlorobis[bis(diphenylphosphino-η⁵-cyclopentadienyl)dichlorotitan(lV)-κ²P,P']-rhodium(I)
   Dichlorobis[(1-diphenylphosphino)-η⁵-indenyl]dichlorozirconium(lV)-κ²P,P']-nickel(II)
Dichloro[1,2-ethylen-bis(1-diphenylphosphino)-η⁵-indenyl]dichlorozirconium(IV)-κ²P,P']-nickel(II)
   Carbonylhydrido[1,2-ethylen-bis(diphenylphosphino-η⁵-cyclopentadienyl)dichlorozirconium(lV)-κ²P,P']triphenylphosphinorhodium(I)
   Carbonylchloro[dimethyldisilandiyl-bis(3-diphenylphosphino-η⁵-cyclopentadienyl)chloro-µ-chloro-zirconium(IV)-K2P,P']rhodium(l)
   Dichloro[dimethylsilandiyl-bis(3-diphenylphosphino-η⁵-indenyl)dichlorozirconium(lV)-κ²P,P']-platin(II)

Die Herstellung der bimetallischen Übergangsmetallverbindungen soll durch die nachfolgenden Syntheseschemata veranschaulicht werden.

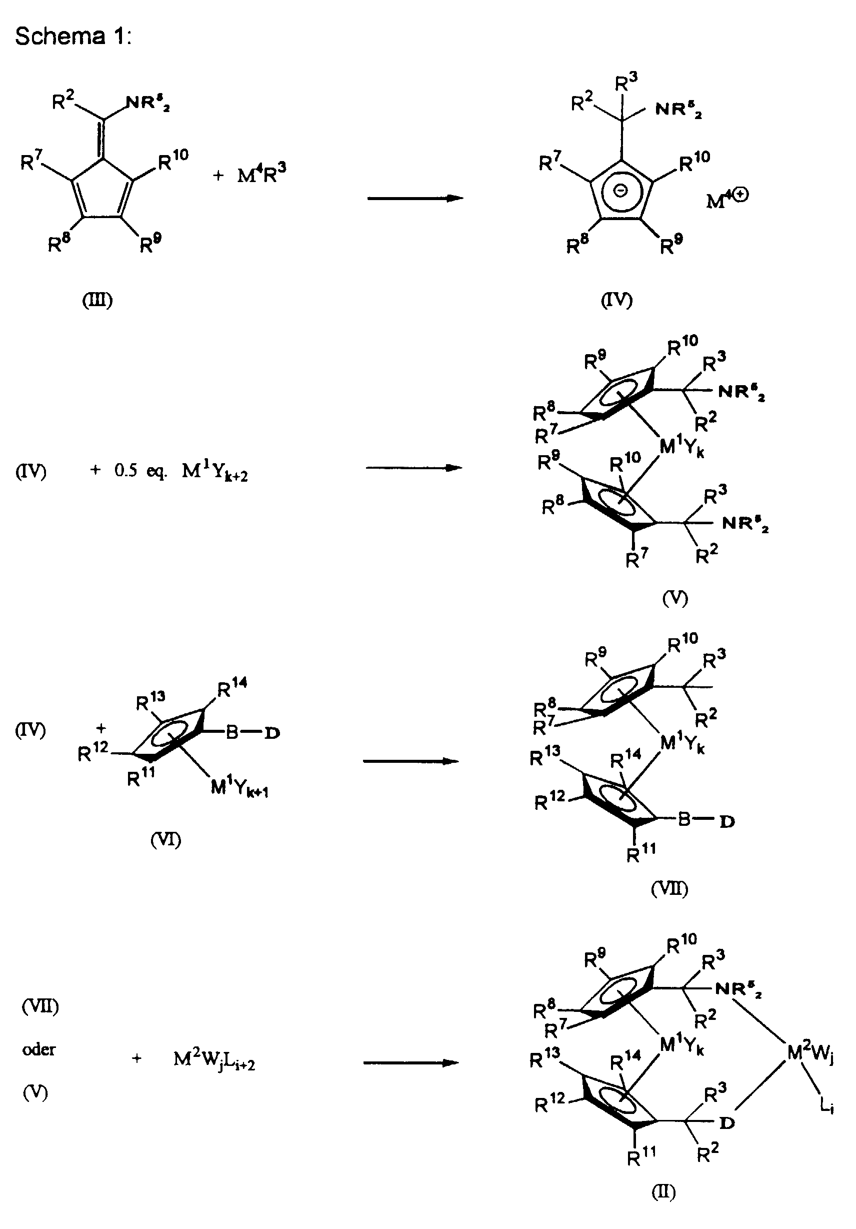

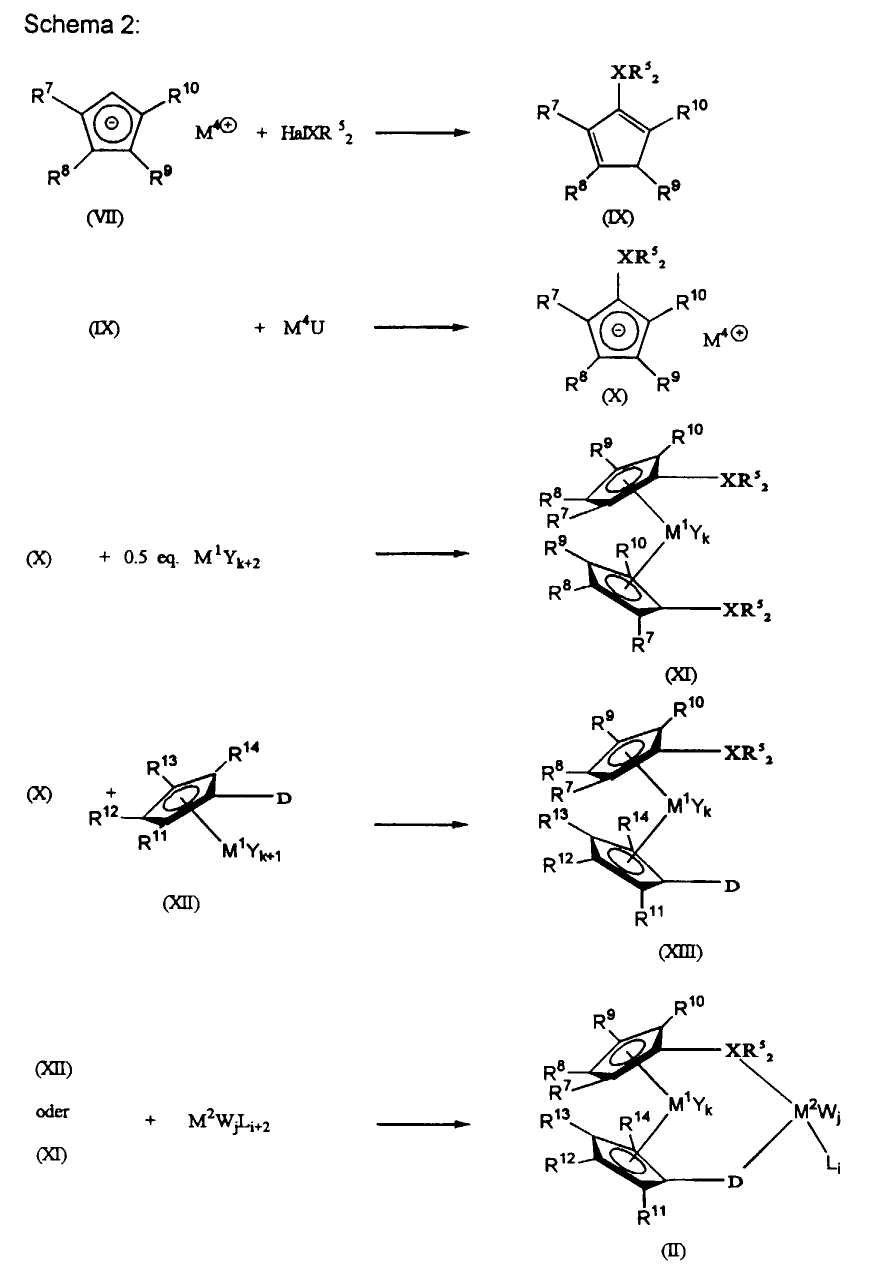

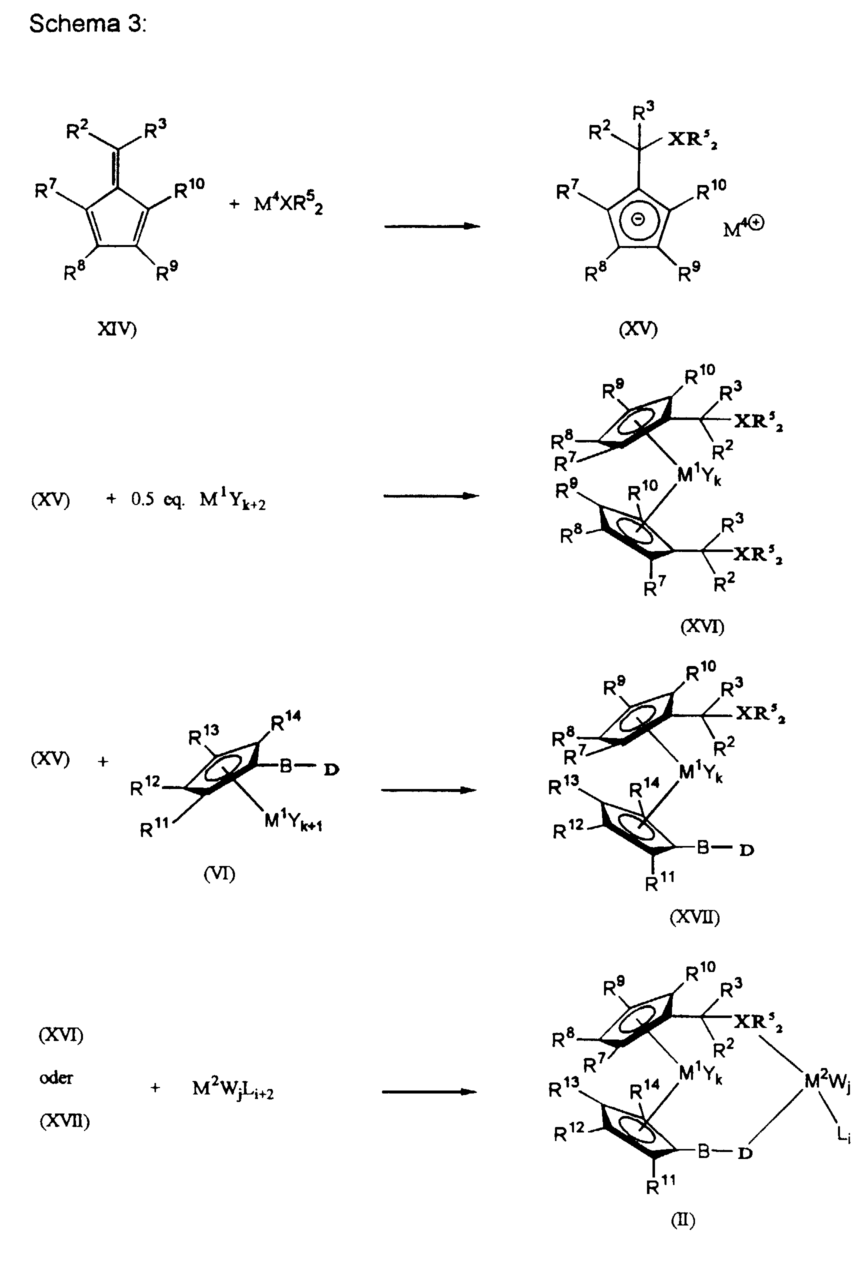

Die Verbindungen der Formel (III), (IX) und (XV) können nach literaturbekannten Methoden hergestellt werden (J. Organomet. Chem. 1986, 310, 335; J. Organomet. Chem. 1988, 353, 93; J. Organomet. Chem. 1987, 320, 349; Organometallics 1982, 1, 1591; Liebigs Ann. Chem. 1964, 39, 678; Angew. Chem. 1980, 92, 1043). Die Umsetzung der Verbindungen der Formel (III) und (IX) zu den gewünschten Verbindungen (IV) und (X) erfolgt mit einer nukleophilen und/oder basischen Verbindung M⁴R³ oder M⁴U, wobei M³ und M⁴ Metalle der Gruppe la oder II a des Periodensystems der Elemente sind, R³ wie in Formel (II) definiert ist und U wie R¹ in Formel (I) definiert ist, und ist im Prinzip bekannt (J. Organomet. Chem 1989, 371, 15; J. Organomet. Chem. 1979, 170, C41; J. Chem. Soc. 1961, 4619). Die Umsetzung der durch diese Reaktion enstehenden Monolithiumverbindung (IV), (X) und (XV) mit dem entsprechenden Metallhalogenid M¹(Y)ₖ₊₂, wobei k eine ganze Zahl von 1 bis 4 ist (z. B.Vanadiumtrichlorid, Zirkoniumtetrachlorid, Niobpentachlorid) ist im Prinzip bekannt und führt zur Bildung der Verbindungen (V), (VII), (Xl), (XIII), (XVI), (XVII) und kann in einem inertem Lösungmittel erfolgen (z. B. J. Organomet. Chem. 1995, 486, 287). Die weitere Umsetzung zu den bimetallischen Übergangsmetallverbindungen der Formel (I) ist im Prinzip bekannt (J. Organomet. Chem. 1995, 494, 261; J. Organomet. Chem. 1982, 231, C43; Organometallics 1988, 7, 2285).

Geeignete inerte Lösungsmittel sind aliphatische oder aromatische Lösemittel, wie beispielsweise Hexan oder Toluol, etherische Lösemittel, wie beispielsweise Tetrahydrofuran oder Diethylether oder halogenierte Kohlenwasserstoffe, wie beispielsweise Methylenchlorid oder halogenierte aromatische Kohlenwasserstoffe wie beispielsweise o-Dichlorbenzol.

Die bimetallischen Übergangsmetallverbindungen der Formel (I) sind hochaktive Katalysatorkomponenten für die Homo- und Copolymerisation von Olefinen und polaren Monomeren und können auch als Katalysatoren in der Hydroformylierung eingesetzt werden.

Die vorliegende Erfindung betrifft sowohl ein Verfahren zur Herstellung eines Olefinpolymers durch Polymerisation eines oder mehrerer Olefine in Gegenwart eines Katalysators, enthaltend mindestens eine bimetallische Übergangsmetallverbindung der Formel I und mindestens einen Cokatalysator wie auch ein Verfahren zur Hydroformylierung von Olefinen und Monoolefinen, nichtkonjugierten Polyolefinen, Cycloolefinen oder Derviaten dieser Verbindungsklassen mit Kohlenmonoxid und Wasserstoff in Gegenwart einer bimetallischen Übergangsmetallverbindung der Formel (I).

Bevorzugt werden in dem erfindungsgemäßen Polymerisationsverfahren eines oder mehrere Olefine der Formel R^{a}-CH=CH-R^{b} homo- oder copolymerisiert, worin R^{a} und R^{b} gleich oder verschieden sind und ein Wasserstoffatom oder einen Kohlenwasserstoffrest mit 1 bis 20 C-Atomen, insbesondere 1 bis 10 C-Atomen, bedeuten, oder R^{a} und R^{b} zusammen mit den sie verbindenen Atomen einen oder mehrere Ringe bilden. Beispiele für solche Olefine sind 1-Olefine wie Ethylen, Propylen, 1-Buten, 1-Penten, 1-Hexen, 4-Methyl-1-penten oder 1-Octen, Styrol, cyclische und acyclische Diene wie 1,3-Butadien, Isopren, 1,4-Hexadien, Norbornadien, Vinylnorbomen oder 5-Ethylidennorbornen. Bevorzugt werden in dem erfindungsgemäßen Verfahren Ethylen oder Propylen homopolymerisiert, oder Ethylen und Propylen werden miteinander und/oder mit einem oder mehreren 1-Olefinen mit 4 bis 20 C-Atomen und/oder einem oder mehreren Dienen mit 4 bis 20 C-Atomen, wie 1,3-Butadien, copolymerisiert.

Die Polymerisation wird bevorzugt bei einer Temperatur von -60 bis 250°C, besonders bevorzugt 50 bis 200°C, durchgeführt. Der Druck beträgt bevorzugt 0,5 bis 2000 bar, besonders bevorzugt 5 bis 64 bar.

Die Polymerisation kann in Lösung, in Masse, in Suspension oder in der Gasphase, kontinuierlich oder diskontinuierlich, ein- oder mehrstufig durchgeführt werden. Eine bevorzugte Ausführungsform ist die Suspensions- und die Gasphasenpolymerisation.

Bevorzugt enthält der in dem erfindungsgemäßen Verfahren eingesetzte Katalysator eine Übergangsmetallverbindung. Es können auch Mischungen zweier oder mehrerer Übergangsmetallverbindungen eingesetzt werden, z.B. zur Herstellung von Polyolefinen mit breiter oder multimodaler Molmassenverteilung.

Prinzipiell ist als Cokatalysator in dem erfindungsgemäßen Verfahren jede Verbindung geeignet, die aufgrund ihrer Lewis-Acidität die neutrale Übergangsmetallverbindung in ein Kation überführen und dieses stabilisieren kann ("labile Koordination"). Darüber hinaus soll der Cokatalysator oder das aus ihm gebildete Anion keine weiteren Reaktionen mit dem gebildeten Übergangsmetallverbindungskation eingehen (EP 427 697). Als Cokatalysator wird bevorzugt eine Aluminiumverbindung und/oder eine Borverbindung verwendet.

Die Borverbindung hat bevorzugt die Formel R¹⁵ₓNH₄₋ₓBR¹⁶₄, R¹⁵ₓPH₄₋ₓBR¹⁶₄, R¹⁵₃CBR¹⁶₄ oder BR¹⁶₃, worin x eine Zahl von 1 bis 4, bevorzugt 3, bedeutet, die Reste R²⁰ gleich oder verschieden, bevorzugt gleich sind, und C₁-C₁₀-Alkyl oder C₆-C₁₈-Aryl sind, oder zwei Reste R¹⁵ zusammen mit dem sie verbindenden Atomen einen Ring bilden, und die Reste R¹⁶ gleich oder verschieden, bevorzugt gleich sind, und C₆-C₁₈-Aryl sind, das durch Alkyl, Haloalkyl oder Fluor substituiert sein kann. Insbesondere steht R¹⁵ für Ethyl, Propyl, Butyl oder Phenyl und R¹⁶ für Phenyl, Pentafluorophenyl, 3,5-Bistrifluoromethylphenyl, Mesityl, Xylyl oder Tolyl (EP 277 003, EP 277 004 und EP 426 638).

Bevorzugt wird als Cokatalysator eine Aluminiumverbindung wie Aluminoxan und/oder ein Aluminiumalkyl eingesetzt.

Besonders bevorzugt wird als Cokatalysator ein Aluminoxan, insbesondere der Formel XVllla für den linearen Typ und/oder der Formel XVlllb für den cyclischen Typ verwendet, 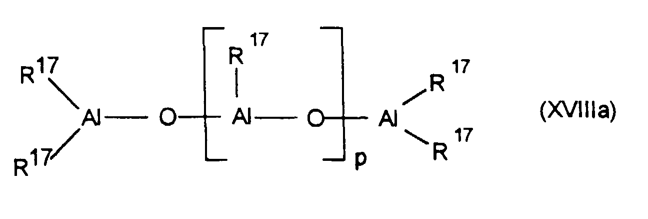 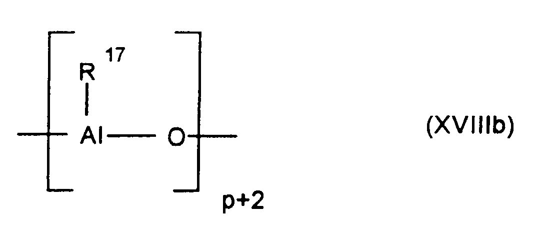 wobei in den Formeln XVllla und XVlllb die Reste R¹⁷ gleich oder verschieden sind und Wasserstoff oder eine C₁-C₂₀-Kohlenwasserstoffgruppe wie eine C₁-C₁₈-Alkylgrupppe, eine C₆-C₁₈-Arylgruppe wie Phenyl oder Benzyl bedeuten, und p eine ganze Zahl von 2 bis 50, bevorzugt 10 bis 35 bedeutet.

Bevorzugt sind die Reste R¹⁷ gleich und bedeuten Wasserstoff, Methyl, Isobutyl, Phenyl oder Benzyl, besonders bevorzugt Methyl.

Sind die Reste R¹⁷ verschieden, so sind sie bevorzugt Methyl und Wasserstoff oder alternativ Methyl und Isobutyl, wobei Wasserstoff oder Isobutyl bevorzugt in einem zahlenmäßigen Anteil von 0,01 bis 40 % (der Reste R¹⁷) enthalten sind.

Die Verfahren zur Herstellung der Aluminoxane sind bekannt. Die genaue räumliche Stuktur der Aluminoxane ist nicht bekannt (J. Am. Chem. Soc. (1993) 115, 4971). Beispielsweise ist denkbar, daß sich Ketten und Ringe zu größeren zweidimensionalen oder dreidimensionalen Strukturen verbinden.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, die Übergangsmetallverbindung vor dem Einsatz in der Polymerisationsreaktion mit einem Cokatalysator, insbesondere einem Aluminoxan vorzuaktivieren. Dadurch wird die Polymerisationsaktivität deutlich erhöht. Die Voraktivierung der Übergangsmetallverbindung wird vorzugsweise in Lösung vorgenommen. Bevorzugt wird dabei die Übergangsmetallverbindung in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösungsmenge. Die Übergangsmetallverbindung kann in der gleichen Konzentration eingesetzt werden, vorzugsweise wird es jedoch in einer Menge von 10⁻⁴ bis 1 mol pro mol Aluminoxan eingesetzt. Die Voraktivierungsdauer beträgt 5 Minuten bis 60 Stunden, vorzugsweise 5 bis 60 Minuten. Man arbeitet bei einer Temperatur von -78 bis 150°C, vorzugsweise 0 bis 80°C.

Die Übergangsmetallverbindung wird bevorzugt in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan, wird bevorzugt in einer Konzentration von 10⁻⁶ bis 10⁻¹ mol, vorzugsweise 10⁻⁵ bis 10⁻² mol pro dm³ Lösemittel bzw. pro dm³ Reaktorvolumen verwendet. Die anderen genannten Cokatalysatoren werden in etwa äquimolaren Mengen zu der Übergangsmetallverbindung verwendet. Prinzipiell sind aber auch höhere Konzentrationen möglich.

Das Aluminoxan kann auf verschiedene Arten nach bekannten Verfahren hergestellt werden. Eine der Methoden ist beispielsweise, daß eine Aluminiumkohlenwasserstoffverbindung und/oder ein Hydridoaluminiumkohlenwasserstoffverbindung mit Wasser (gasförmig, fest, flüssig oder gebunden - beispielsweise als Kristallwasser) in einem inerten Lösungsmittel (wie beispielsweise Toluol) umgesetzt wird. Zur Herstellung eines Aluminoxans mit verschiedenen Resten R¹⁷ werden beispielsweise entsprechend der gewünschten Zusammensetzung zwei verschiedene Aluminiumtrialkyle mit Wasser umgesetzt.

Zur Entfernung von im Olefin vorhandenen Katalysatorgiften ist eine Reinigung mit einer Aluminiumverbindung, bevorzugt einem Aluminiumalkyl, wie Trimethylaluminium oder Triethylaluminium, vorteilhaft. Diese Reinigung kann sowohl im Polymerisationssystem selbst erfolgen, oder das Olefin wird vor der Zugabe in das Polymerisationssystem mit der Aluminiumverbindung in Kontakt gebracht und anschließend wieder abgetrennt.

Als Molmassenregler und/oder zur Steigerung der Katalysatoraktivität kann in dem erfindungsgemäßen Verfahren Wasserstoff zugegeben werden. Hierdurch können niedermolekulare Polyolefine wie Wachse erhalten werden.

Bevorzugt wird in dem erfindungsgemäßen Verfahren die Übergangsmetallverbindung mit dem Cokatalysator außerhalb des Polymerisationsreaktors in einem separaten Schritt unter Verwendung eines geeigneten Lösemittels umgesetzt. Dabei kann eine Trägerung vorgenommen werden.

Im dem erfindungsgemäßen Verfahren kann mit Hilfe der Übergangsmetallverbindung eine Vorpolymerisation erfolgen. Zur Vorpolymerisation wird bevorzugt das (oder eines der) in der Polymerisation eingesetzte(n) Olefin(e) verwendet.

Der in dem erfindungsgemäßen Verfahren eingesetzte Katalysator kann geträgert sein. Durch die Trägerung läßt sich beispielsweise die Kornmorphologie des hergestellten Polyolefins steuern. Dabei kann die Übergangsmetallverbindung zunächst mit dem Träger und anschließend mit dem Cokatalysator umgesetzt werden. Es kann auch zunächst der Cokatalysator geträgert werden und anschließend mit der Übergangsmetallverbindung umgesetzt werden. Auch ist es möglich das Reaktionsprodukt von Übergangsmetallverbindung und Cokatalysator zu trägem. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form. Die Herstellung eines geträgerten Cokatalysators kann beispielsweise wie in EP 567 952 beschrieben durchgeführt werden.

Vorzugsweise wird der Cokatalysator, z.B. Aluminoxan, auf einen Träger wie beispielsweise Silikagele, Aluminiumoxide, festes Aluminoxan, andere anorganische Trägermaterialien oder auch ein Polyolefinpulver in feinverteilter Form aufgebracht und dann mit der Übergangsmetallverbindung umgesetzt.

Als anorganische Träger können Oxide eingesetzt werden, die flammenpyrolytisch durch Verbrennung von Element-Halogeniden in einer Knallgas-Flamme erzeugt wurden, oder als Kieselgele in bestimmten Korngrößen-Verteilungen und Komformen herstellbar sind.

Hierdurch können niedermolekulare Polyolefine wie Wachse erhalten werden.

Bevorzugt wird in dem erfindungsgemäßen Verfahren die Übergangsmetallverbindung mit dem Cokatalysator außerhalb des Polymerisationsreaktors in einem separaten Schritt unter Verwendung eines geeigneten Lösemittels umgesetzt. Dabei kann eine Trägerung vorgenommen werden.

Im dem erfindungsgemäßen Verfahren kann mit Hilfe der Übergangsmetallverbindung eine Vorpolymerisation erfolgen. Zur Vorpolymerisation wird bevorzugt das (oder eines der) in der Polymerisation eingesetzte(n) Olefin(e) verwendet.

Der in dem erfindungsgemäßen Verfahren eingesetzte Katalysator kann geträgert sein. Durch die Trägerung läßt sich beispielsweise die Kornmorphologie des hergestellten Polyolefins steuern. Dabei kann die Übergangsmetallverbindung zunächst mit dem Träger und anschließend mit dem Cokatalysator umgesetzt werden. Es kann auch zunächst der Cokatalysator geträgert werden und anschließend mit der Übergangsmetallverbindung umgesetzt werden. Auch ist es möglich das Reaktionsprodukt von Übergangsmetallverbindung und Cokatalysator zu trägern. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form. Die Herstellung eines geträgerten Cokatalysators kann beispielsweise wie in EP 567 952 beschrieben durchgeführt werden.

Vorzugsweise wird der Cokatalysator, z.B. Aluminoxan, auf einen Träger wie beispielsweise Silikagele, Aluminiumoxide, festes Aluminoxan, andere anorganische Trägermaterialien oder auch ein Polyolefinpulver in feinverteilter Form aufgebracht und dann mit der Übergangsmetallverbindung umgesetzt.

Als anorganische Träger können Oxide eingesetzt werden, die flammenpyrolytisch durch Verbrennung von Element-Halogeniden in einer Knallgas-Flamme erzeugt wurden, oder als Kieselgele in bestimmten Korngrößen-Verteilungen und Kornformen herstellbar sind.

Die Herstellung eines geträgerten Cokatalysators kann beispielsweise wie in EP 578 838 beschrieben in der folgenden Weise in einem Edelstahl-Reaktor in explosionsgeschützter Ausführung mit einem Umpumpsystem der Druckstufe 60 bar, mit Inertgasversorgung, Temperierung durch Mantelkühlung und zweitem Kühlkreislauf über einen Wärmetauscher am Umpumpsystem erfolgen. Das Umpumpsystem saugt den Reaktorinhalt über einen Anschluß im Reaktorboden mit einer Pumpe an und drückt ihn in einen Mischer und durch eine Steigleitung über einen Wärmetauscher in den Reaktor zurück. Der Mischer ist so gestaltet, daß sich in dem Zulauf ein verengter Rohrquerschnitt befindet, wo eine erhöhte Strömungsgeschwindigkeit entsteht, und in dessen Turbulenzzone axial und entgegen der Strömungsrichtung eine dünne Zuleitung geführt ist, durch welche - getaktet - jeweils eine definierte Menge Wasser unter 40 bar Argon eingespeist werden kann. Die Kontrolle der Reaktion erfolgt über einen Probennehmer am Umpumpkreislauf. Im Prinzip sind auch andere Reaktoren geeignet.

Weitere Möglichkeiten der Herstellung eines geträgerten Cokatalysators sind in EP 578 838 beschrieben. Danach wird die erfindungsgemäße Übergangsmetallverbindung auf den geträgerten Cokatalysator aufgebracht, indem die gelöste Übergangsmetallverbindung mit dem geträgerten Cokatalysator gerührt wird. Das Lösemittel wird entfernt und durch einen Kohlenwasserstoff ersetzt, in dem sowohl Cokatalysator als auch die Übergangsmetallverbindung unlöslich sind.

Die Reaktion zu dem geträgerten Katalysatorsystem erfolgt bei einer Temperatur von -20 bis +120°C, bevorzugt 0 bis 100°C, besonders bevorzugt bei 15 bis 40°C. Die Übergangsmetallverbindung wird mit dem geträgerten Cokatalysator in der Weise umgesetzt, daß der Cokatalysator als Suspension mit 1 bis 40 Gew.-%, bevorzugt mit 5 bis 20 Gew.-% in einem aliphatischen, inerten Suspensionsmittel wie n-Decan, Hexan, Heptan, Dieselöl mit einer Lösung der Übergangsmetallverbindung in einem inerten Lösungsmittel wie Toluol, Hexan, Heptan, Dichlormethan oder mit dem feingemahlenen Feststoff des Übergangsmetallverbindung zusammengebracht wird. Umgekehrt kann auch eine Lösung der Übergangsmetallverbindung mit dem Feststoff des Cokatalysators umgesetzt werden.

Die Umsetzung erfolgt durch intensives Mischen, beispielsweise durch Verrühren bei einem molaren Al/M¹-Verhältnis von 100/1 bis 10000/1, bevorzugt von 100/1 bis 3000/1 sowie einer Reaktionszeit von 5 bis 120 Minuten, bevorzugt 10 bis 60 Minuten, besonders bevorzugt 10 bis 30 Minuten unter inerten Bedingungen. Im Laufe der Reaktionszeit zur Herstellung des geträgerten Katalysatorsystems treten insbesondere bei der Verwendung der erfindungsgemäßen Übergangsmetallverbindungen mit Absorptionsmaxima im sichtbaren Bereich Veränderungen in der Farbe der Reaktionsmischung auf, an deren Verlauf sich der Fortgang der Reaktion verfolgen läßt.

Nach Ablauf der Reaktionszeit wird die überstehende Lösung abgetrennt, beispielsweise durch Filtration oder Dekantieren. Der zurückbleibende Feststoff wird 1- bis 5-mal mit einem inerten Suspensionsmittel wie Toluol, n-Decan, Hexan, Dieselöl, Dichlormethan zur Entfernung löslicher Bestandteile im gebildeten Katalysator, insbesondere zur Entfernung von nicht umgesetzter und damit löslicher Übergangsmetallverbindung, gewaschen.

Das so hergestellte geträgerte Katalysatorsystem kann im Vakuum getrocknet als Pulver oder noch Lösemittel behaftet wieder resuspendiert und als Suspension in einem der vorgenannten inerten Suspensionsmittel in das Polymerisationssystem eindosiert werden.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Hexan, Heptan, Isooctan, Cyclohexan, Methylcyclohexan, genannt. Weiterhin kann eine Benzin- bzw. hydrierte Dieselölfraktion benutzt werden. Brauchbar ist auch Toluol. Bevorzugt wird im flüssigen Monomeren polymerisiert.

Vor der Zugabe des Katalysators, insbesondere des geträgerten Katalysatorsystems (aus einer erfindungsgemäßen Übergangsmetallverbindung und einem geträgerten Cokatalysator beziehungsweise aus einer erfindungsgemäßen Übergangsmetallverbindung und einer aluminiumorganischen Verbindung auf einem Polyolefinpulver in feinverteilter Form), kann zusätzlich eine andere Aluminiumalkylverbindung wie beispielsweise Trimethylaluminium, Triethylaluminium, Triisobutylaluminium, Trioctylaluminium oder Isoprenylaluminium zur Inertisierung des Polymerisationssystems (beispielsweise zur Abtrennung vorhander Katalysatorgifte im Olefin) in den Reaktor gegeben werden. Diese wird in einer Konzentration von 100 bis 0,01 mmol Al pro kg Reaktorinhalt dem Polymerisationssystem zugesetzt. Bevorzugt werden Triisobutylaluminium und Triethylaluminium in einer Konzentration von 10 bis 0,1 mmol Al pro kg Reaktorinhalt. Dadurch kann bei der Synthese eines geträgerten Katalysatorsystems das molare Al/M¹-Verhältnis klein gewählt werden.

Werden inerte Lösemittel verwendet, werden die Monomeren gasförmig oder flüssig zudosiert.

Die Dauer der Polymerisation ist beliebig, da das in dem erfindungsgemäßen Verfahren zu verwendende Katalysatorsystem einen nur geringen zeitabhängigen Abfall der Polymerisationsaktivität zeigt.

Die bimetallische Übergangsmetallverbindung kann (ohne Cokatalysator) in der Hydroformylierung von Olefinen mit Kohlenmonoxid und Wasserstoff eingesetzt werden.

Bevorzugt wird in dem erfindungsgemäßen Hydroformylierungsverfahren ein Olefin der Formel R^{a}-CH=CH-R^{b}, worin R^{a} und R^{b} gleich oder verschieden sind und ein Wasserstoffatom oder einen Kohlenwasserstoffrest mit 1 bis 20 C-Atomen, inbesondere 1 bis 10 C-Atomen, bedeuten, oder R^{a} und R^{b} zusammen mit den sie verbindenen Atomen einen oder mehrere Ringe bilden, mit Kohlenmonoxid und Wasserstoff zu einem Alkohol oder Aldehyd umgesetzt, welcher ein Kohlenstoffatom mehr als im Ausgangsolefin enthält. Beispiele für solche Olefine sind Ethylen, Propylen, 1-Buten, 2-Buten, 1-Penten, 2-Methylbuten, 1-Hexen, 2-Hexen, 1-Hepten, 1-Octen, 3-Octen, 3-Ethylhexen-1, 1-Decen, 3-Undecen, 4,4-Dimethylnonen-1, Dicyclopentadien, Vinylcyclohexen, Cyclooctadien, 4-Methyl-1-penten, Styrol, cyclische und acyclische Diene wie 1,3-Butadien, Isopren, 1,4-Hexadien, Norbornadien, Vinylbornen oder 5-Ethylidennorbomen. Derivate der genannten Olefinarten, die durch die bimetallischen Übergangsmetallverbindung der allgemeinen Formel (I) ebenfalls hydroformyliert werden können sind z.B. Alkohole, Aldehyde, Carbonsäuren, Ester, Nitrile, Halogenverbindungen, Allylalkohole, Acrolein, Methacrolein, Crotonalaldehyd, Methylacrylat, Ethylcrotonat, Diethylfumarat, Diethylmaleinat und Acrylnitril.

Die Reaktion wird durch eine Verbindung der Formel (I) katalysiert, welche vorzugsweise Metalle der Gruppe VIII b des Periodensystems der Elemente enthält. Neben Cobalt, das als Katalysatormetall in großem Umfang technische Anwendung findet, gewinnt in letzter Zeit Rhodium zunehmend Bedeutung. Im Gegensatz zu Cobalt gestattet Rhodium, die Reaktion bei niedrigem Druck durchzuführen; darüber hinaus werden vorzugsweise geradkettige n-Aldehyde und nur in untergeordnetem Masse iso-Aldehyde gebildet. Schließlich ist auch die Hydrierung der Olefine zu gesättigten Kohlenwasserstoffen in Gegenwart von Rhodium-Katalysatoren deutlich niedriger als bei Anwendung von Cobalt-Katalysatoren.
Die Lösung oder Suspension des Katalysators in einem inertem Lösungsmittel wird entweder im Hydroformylierungsreaktor oder vorab, in einer separaten Vorrichtung hergestellt und darauf dem Hydroformylierungsreaktor zugeleitet. Die Konzentration des Übergangsmetalls M² beträgt 20 bis 1000-Gew.-ppm (bezogen auf die Lösung), vorzugsweise 100 bis 600 Gew.-ppm. und insbesondere 200 bis 400 Gew.-ppm. Die Umsetzung des Olefins mit Kohlenmonoxid und Wasserstoff erfolgt bei Drücken von etwa 0,1 bis etwa 30 MPa, vorzugsweise 1 bis 12 MPa und insbesondere 3 bis 7 MPa.
Die Zusammensetzung des Synthesesgases d.h. das Volumenverhältnis von Kohlenmonoxid und Wasserstoff kann sich über weite Bereiche erstrecken und z. B. zwischen 1 : 10 bis 10 : 1 varriert werden. Im Allgemeinen setzt man Gasgemische ein, in denen das Volumenverhältnis von Kohlenmonoxid und Wasserstoff etwa 1 : 1 ist oder von diesem Wert in der einen oder anderen Richtung nur wenig abweicht. Die Reaktionstemperatur liegt zwischen etwa 20 und 150°C, bevorzugt zwischen werden zwischen 80 und 140°C und insbesondere bevorzugt zwischen 100 und 125°C.
Die Umsetzung der in flüssiger und gasförmiger Phase vorliegenden
Reaktionspartner erfolgt in konventionellen Reaktoren. Der Ablauf der Umsetzung wird maßgeblich dadurch beeinflußt, daß die Katalysatorlösung mit dem flüssigen oder gasförmigen Olefin und dem Synthesegas gesättigt werden muß. Daher ist es erforderlich, eine möglichst große Berührungsfläche zwischen den Phasen zu erzeugen. Es hat sich bewährt, den flüssigen Reaktorinhalt intensiv zu rühren und die gasförmigen Reaktionspartner über Verteilungsvorrichtungen der flüssigen Phase zuzuführen.

### Beispiele:

### A: Synthese von bimetallischen Übergangsmetallverbindungen mit diphenylphosphinosubstituierten Metallocendichloriden

### Beispiel A1: cis-Dichlorobis[(diphenylphosphino-η⁵-cyclopentadienyl)dichlorozirconium-(IV)-κ²P,P']-palladium(II)

Diphenylphosphinocyclopentadien
Zu einer Lösung von 940 mg (10.7 mmol) Cyclopentadienylnatrium (NaCp) in 30 ml THF bei -78 °C 1.96 ml (2.36 g, 10.7 mmol) Chlorodiphenylphosphan langsam zugetropft. Es wird 2 h bei Raumtemperatur gerührt und dann über 2 cm Celite auf einer Glasfritte filtriert. Die gelbe Lösung wird im Ölpumpenvakuum eingeengt und das zurückbleibende orangefarbene Öl direkt weiter umgesetzt.
Ausbeute: 2.20 g (82 %)
¹H-NMR (CDCl₃, 200 MHz): δ = 7.4-7.2 (m, 20H, Ph-H), 6.68-6.40 (m, 6H, Cp-H), 3.10 (m, 2H, Cp-H, CH₂), 2.98 (m, 2H, Cp-H, CH₂) ppm.

(Diphenylphosphinocyclopentadienyl)lithium
2.20 g (8.79 mmol) Diphenylphosphinocyclopentadien werden in Toluol gelöst und bei -78 °C 6.65 ml 1.6 M n-Butyllithium (1.2 Äquivalente, 10.6 mmol) zugetropft. Es wird über Nacht bei Raumtemperatur gerührt und der ausgefallene, schwach gelbe Feststoff auf eine Fritte überführt, dort mit 10 ml Pentan zweimal nachgewaschen und das weiße Pulver im Ölpumpenvakuum getrocknet.
Ausbeute: 2.02 g (90 %)
¹H-NMR (Benzol-d₆/THF-d₈, 200 MHz): δ = 7.56-7.48 (m, 4H, o-Ph-H), 7.12-7.00 (m, 6H, m-Ph-H u. p-Ph-H), 6.3-6.2 (m, 4H, Cp-H) ppm.

Bis(diphenylphosphino-η⁵-*cyclopentadienyl)dichlorozirconium*
Es werden 7.68 g (30 mmol) Diphenylphosphinocyclopentadienyllithium und 5.66 g Bis(tetrahydrofuran)tetrachlorozirconium(IV) (15mmol) fest gemischt und bei -78 °C 300 ml Toluol unter kräftigem Rühren zugesetzt. Es wird bei Raumtemperatur über Nacht gerührt, filtriert und das Lösungsmittel im Vakuum entfernt. Reinweißes Produkt wird auf eine Glasfritte überführt und nach dreimaligem Waschen mit je 30 ml Pentan im Ölpumpenvakuum getrocknet.
Ausbeute: 7.9 g (12 mmol)
¹H-NMR (Benzol-d₆, 200 MHz): δ = 7.42-7.29 (m, 8H, o-Ph-H), 7.10-6.95 (m, 12H, m-Ph-H u. p-Ph-H), 6.23-6.19 (m, 4H, 2-H), 6.11-6.08 (m, 4H, 3-H) ppm.

cis-Dichlorobis[(diphenylphosphino-η⁵-cyclopentadienyl)dichlorozirconium(lV)-κ²P,P']-palladium(ll)
572 mg (0.87 mmol) Bis(diphenylphosphino-η⁵-cyclopentadienyl)dichlorozirconium(IV) und 333 mg (0.87 mmol)
Bis(benzonitril)dichloropalladium(II) werden zur Reaktion gebracht. Das schwachgelbe Produkt wird im Ölpumpenvakuum getrocknet.
Ausbeute: 620 mg (0.74 mmol), 85%.
¹H-NMR (CD₂Cl₂, 200 MHz): δ = 7.7-7.5 (m, 12H, Ph-H), 7.45-7.35 (m, 8H, m-Ph-H), 6.92 (m, 4H, Cp-H), 6.34 (m, 4H, Cp-H) ppm.

### Beispiel A2: Dichlorobis[(diphenylphosphino-η⁵-cyclopentadienyl)dichlorozirconium(IV)-κ²P,P']-platin(II)

66 mg Bis(diphenylphosphino-η⁵-cyclopentadienyl)dichlorozirconium (0.1 mmol) und 47 mg PtCl₂(PhCN)₂ (0.1 mmol) werden in THF zur Reaktion gebracht, 4 h gerührt und das Produkt nach Pentanzugabe als nahezu unlöslicher Feststoff isoliert. Ausbeute: 75 mg (0.075 mmol), 75% Mono-THF-Addukt. (C₃₄H₂₈Cl₄P₂PtZr, M = 926.67 g/mol)
¹H-NMR (CD₂Cl₂, 200 MHz): δ = 7.7-7.3 (m, 10H, Ph-H), 6.9 (m, 4H, Cp-H), 6.4 (m, 4H, Cp-H) ppm.

### Beispiel A3: Di-µ-chlorobis[bis(diphenylphosphino-η⁵-cyclopentadienyl)dichlorozirconium(lV)-κ²P,P']-rhodium(I)

Bei -30 °C werden 440 mg (0.66 mmol) Bis(diphenylphosphino-η⁵-cyclopentadienyl)dichlorozirconium(IV) in 30 ml CH₂Cl₂ zu 164 mg (0.33 mmol) Cyclooctadien-µ-chlororhodium(l)-Dimer in 20 ml CH₂Cl₂ getropft. Die klare Lösung färbt sich dunkelrot. Nach 6 h wird das Dichlormethan bis auf 5 ml entfernt und 30 ml Pentan zugegeben. Das orangerote Produkt wird dreimal mit je 5 ml Pentan gewaschen und im Ölpumpenvakuum getrocknet.
Ausbeute: 475 mg (90 %, 0.30 mmol)
¹H-NMR (CD₂Cl₂, 200 MHz): δ = 7.46 (m, 16H, Ph-H), 7.27 (pt, 8H, Ph-H, J = 7.3 Hz), 7.09 (pt, 16H, Ph-H, J = 7.3 Hz), 6.74 (m, 8H, Cp-H), 6.35 (m, 8H, Cp-H) ppm.

### Beispiel A4: Carbonylchloro[bis(diphenylphosphino-η⁵-cyclopentadienyl)chloro-µ-chloro-zirconium(IV)-κ²P,P']rhodium(I)

340 mg Bis(diphenylphosphino-η⁵-cyclopentadienyl)dichlorozirconium(IV) (0.51 mmol) und 100 mg Bis[dicarbonyl-µ-chlororhodium] (0.254 mmol) werden getrennt in je 30 ml CH₂Cl₂ gelöst. Bei 0 °C wird die Zirconiumverbindung zum Rhodiumkomplex getropft und die Reaktionsmischung 24 h bei Raumtemperatur gerührt. Das Dichlormethan wird bis auf 5 ml im Vakuum entfernt und die Fällung mit 20 ml Pentan vervollständigt. Das gelbe Produkt wird auf einer Fritte isoliert und im Ölpumpenvakuum getrocknet. Die sehr schlecht lösliche Verbindung konnte aus THF in Form röntgenfähiger Kristalle umkristallisiert werden.
Ausbeute: 375 mg (0.45 mmol), 89 %.
¹H-NMR (CD₂Cl₂, 200 MHz): 8.26, 8.11 (je m, je 4H, Ph-H), 7.5 (br m, 12H, Ph-H), 6.60, 6.45, 6.05, 5.84 (je m, je 2H, Cp-H) ppm.

### Beispiel A5: Carbonylhydrido[bis(diphenylphosphino-η⁵-cyclopentadienyl)dichlorozirconium(lV)-κ²P,P']triphenylphosphinorhodium(I)

92 mg (0.1 mmol) Hydridocarbonyltris(triphenylphosphan)rhodium(I) und 66 mg (0.1 mmol) Bis(diphenylphosphino-η⁵-cyclopentadienyl)dichlorozirconium(IV) werden in CH₂Cl₂ getrennt gelöst und bei 0 °C zusammengegeben. Nach 10 min Rühren wird der Großteil des Lösungsmittels entfernt und die Fällung durch Pentanzugabe vervollständigt.
¹H-NMR (Toluol-d₈, CD₂CI₂, 600 MHz): 6.21, 6.15, 6.09, 5.92 (je m, je 2H, Cp-H),-9.86 (ddt, 1H, Rh-H, J = ) ppm.

### Beispiel A6: Di-µ-chlorobis[bis(diphenylphosphino-η⁵-cyclopentadienyl)dichlorotitan(IV)-κ²P,P']-rhodium(I)

Bis(diphenylphosphino-η⁵-*cyclopentadienyl)dichlorotitan* Es werden 3.46 g (13.5 mmol) Diphenylphosphinocyclopentadienyllithium und 2.26 g Bis(tetrahydrofuran)tetrachlorotitan(IV) (6.75 mmol) fest gemischt und bei 0 °C 150 ml Toluol unter kräftigem Rühren zugesetzt. Es wird bei Raumtemperatur 1 h gerührt, dann 1 h auf 80 °C erhitzt, filtriert und das Lösungsmittel im Vakuum entfernt. Das tiefrotbraune Rohprodukt wird in Pentan aufgerührt, auf eine Glasfritte überführt und nach dreimaligem Waschen mit je 10 ml kaltem Diethylether im Ölpumpenvakuum getrocknet.
Ausbeute: 3.10 g (75 %; Lit.: 65 %).
¹H-NMR (CDCl₃, 200 MHz): δ = 7.32 (m, 20H, Ph-H), 6.5-6.4 (br m, 8H, 2-H, 3-H) ppm.

### Di-µ-chlorobis[bis(diphenylphosphino-η⁵-cyclopentadienyl)dichlorotitan(lV)-κ²P,P']rhodium(I)

Bei -78 °C werden 207 mg (0.33 mmol) Bis(diphenylphosphino-η⁵-cyclopentadienyl)dichlorotitan(IV) in 30 ml Toluol zu 82 mg (0.17 mmol) Cyclooctadien-µ-chlororhodium(l)-Dimer in 20 ml Toluol getropft. Nach 24 h wird das Toluol im Vakuum bis auf 20 ml entfernt und 30 ml Pentan zugegeben. Das hellbraune Produkt wird zweimal mit je 5 ml Pentan gewaschen und im Ölpumpenvakuum getrocknet.
Ausbeute: 180 mg (70%, 0.12 mmol) (C₆₈H₅₆CI₆P₄Rh₂Ti₂, M = 1511.38 g/mol)
¹H-NMR (CD₂Cl₂, 200 MHz): δ = 7.46 (m, 16H, Ph-H), 7.29 (m, 8H, Ph-H), 7.11 (m, 16H, Ph-H), 6.87 (m, 8H, Cp-H), 6.37 (m, 8H, Cp-H) ppm.

### Beispiel A7: Dichlorobis[(1-diphenylphosphino)-η⁵-indenyl]dichlorozirconium(lV)-κ²P,P']-nickel(II)

### 1 -Diphenylphosphinoinden

Bei -78 °C werden zu einer Lösung von 2.0 ml (1.97 g, 17.0 mmol) frisch destillierten Indens in 30 ml Tetrahydrofuran 10.6 ml (17.0 mmol), 1.6 M n-Butyllithium-Lösung getropft. Es wird auf Raumtemperatur erwärmt und zwei Stunden gerührt. Nach Abkühlen auf -70 °C werden langsam 3.13 ml (17.0 mmol) Chlorodiphenylphosphan zugegeben. Es wird noch 90 min bei Raumtemperatur gerührt und dann das Lösungsmittel im Ölpumpenvakuum entfernt. Nach Zugabe von 40 ml Toluol und Filtration über Celite wird die Lösung direkt weiter umgesetzt. NMR-Kontrolle zeigt vollständigen Umsatz zu einem Gemisch aus zwei Isomeren.
¹H-NMR (Benzol-d₆, 200 MHz): Isomer a, Isomer b: δ = 7.55-6.85 (3x m, 28H, Ph-H und Ind-H); Isomer a: 6.67 (m, 1H, 3-H), 6.32 (m, 1H, 2-H), 4.4 (br s, 1H, 1-H);
Isomer b: 6.15-6.08 (m, 1H, 2-H), 3.06 (m, 2H, 3-H) ppm.

### 1-(Diphenylphosphino)indenyllithium

Nach Abkühlen der toluolischen Lösung des 1-Diphenylphosphinoindens auf -78 °C werden 13.0 ml (20.8 mmol) 1.6 M n-Butyllithium-Lösung (1.2 Äquivalente) zugetropft und über Nacht gerührt. Der entstandene weiße Feststoff wird isoliert, dreimal mit Pentan gewaschen und im Ölpumpenvakuum getrocknet.
Ausbeute: 2.50 g (49 %)
¹H-NMR (Benzol-d₆/THF-d₈, 200 MHz): δ = 7.97 (m, 1H, 7-H o. 4-H), 7.75 (m, 1H, 4-H o 7-H), 7.66-7.61 (m, 4H, o-Ph-H), 7.12-6.88 (m, 9H, m-Ph-H, p-Ph-H, 5-H, 6-H und 2-H) 6.48 (ddd, 1H, 3-H, ³J_{H-H}, ⁴J_{P-H} = 1.6, 3.6 Hz, ⁵J_{H-H} = 0.8 Hz) ppm.

### Bis[(1-diphenylphosphino)-η⁵-indenyl]dichlorozirconium

925 mg (3.0 mmol) 1-Diphenylphosphinoindenyllithium und 350 mg (1.5 mmol) Zirconiumtetrachlorid werden getrennt in je 20 ml Toluol suspendiert und auf -78 °C abgekühlt. Der Ligand wird zum Metallsalz gegeben. Nach 10 h wird 1 h auf 60 °C erwärmt und filtriert, zweimal mit je 5 ml Toluol nachgewaschen und dann das Lösungsmittel im Ölpumpenvakuum entfernt. Der Rückstand wird in 40 ml Pentan suspendiert, auf einer Glasfritte isoliert, nochmals dreimal mit je 20 ml Pentan gewaschen und Lösungsmittelreste innerhalb von 5 h im Ölpumpenvakuum entfernt. Vollständige Entfernung des Hydrolyseproduktes des Lithiumsalzes gelang bisher nicht. Es entstehen zwei diasteromere Produkte im Verhältnis 1:1 in Form eines gelben Pulvers.
Ausbeute: 830 mg (73 %).
¹H-NMR (Benzol-d₆, 200 MHz): δ = 7.95-6.75 (m, 56H, Ph-H u. Ind-H), 6.05 (dd, 2H, 2-H o. 3-H, J = 3.3 Hz, J = 0.9 Hz), 5.96 (dd, 2H, 3-H o. 2-H, J = 3.3 Hz, J = 0.9 Hz), 5.42 (dd, 2H, 2-H o. 3-H, J = 3.2 Hz, J = 0.7 Hz), 5.15 (br d, 2H, 3-H o. 2-H, J = 3 Hz) ppm.

Dichlorobis[(1-diphenylphosphino)-η⁵-indenyl]dichlorozirconium(lV)-κ²P,P']-nickel(II)
758 mg Bis[(1-diphenylphosphino)-η⁵-indenyl]dichlorozirconium (1.0 mmol) und 335 mg NiCl₂(PhCN)₂ (1.0 mmol) werden in THF zur Reaktion gebracht, 4 h gerührt und das Produkt nach Pentanzugabe als nahezu unlöslicher Feststoff isoliert. Ausbeute: 665 mg (0.075 mmol), 75%
¹H-NMR (Benzol-d₆, 200 MHz): δ = 8.05-6.85 (m, 56H, Ph-H u. Ind-H), 6.02 (dd, 2H, 2-H o. 3-H, J = 3.3 Hz, J = 0.9 Hz), 5.99 (dd, 2H, 3-H o. 2-H, J = 3.3 Hz, J = 0.9 Hz), 5.41 (dd, 2H, 2-H o. 3-H, J = 3.2 Hz, J = 0.7 Hz), 5.15 (br d, 2H, 3-H o. 2-H, J = 3 Hz) ppm.

### B: Darstellung von bimetallischen Übergangsmetallverbindungen mit diphenylphosphinomethylensubstituierten Metallocendichloriden

Beispiel B1: trans-Dichlorobis{[1-(1-methyl-1-diphenylphosphinoethyl)-η⁵-cyclopenta-dienyl]dichlorozirconium(lV)-κ²P,P'}palladium(II) 1 -(1 -Methyl-1-diphenylphosphinoethyl)cyclopentadienyllithium
Zu einer Lösung von 2.09 g (10.7 mmol) Diphenylphosphan in 30 ml THF gibt man bei -10 °C 6.70 ml (10.7 mmol) n-Butyllithium (1.6 M in Pentan). Nach 1 h werden 1.28 ml (1.13 g, 10.7 mmol) 6,6-Dimethylfulven, gelöst in 30 ml Pentan, langsam innerhalb von 1 h zugetropft. Es wird über Nacht gerührt und aufgearbeitet. Ausbeute: 2.40 g (75 %)
¹H-NMR (Benzol-d₆,/THF-d₈, 200 MHz): δ = 7.6-7.0 (2 m, 10H, Verhältnis 4H : 6H, o-Ph-H : m-Ph-H, p-Ph-H), 6.1-5.9 (s, 4H, Cp-H) oder 6.0-5.6 (2 m, je 2H, Cp-H), 3.7-3.4 (s, 1H, α-H, THF-d₇), 1.8-1.4 (s, 1H, β-H,THF-d₇), 1.6-1.3 (d, 6H, ³J_{P-H} = 12.1 Hz, CH₃) ppm.

Bis[1-(1-methyl-1-diphenylphosphinoethyl)-η⁵-*cyclopentadienyl]dichlorozirconium* 1-(1-Methyl-1-diphenylphosphinoethyl)cyclopentadienyllithium (2.21 g, 7.42 mmol) und 1.40 g (3.71 mmol) Zirconiumtetrachlorid x 2 THF werden bei -30 °C zusammengegeben, bei dieser Temperatur noch 4 h gerührt und aufgearbeitet. Es konnten 1.82 g (66 %) des weißen Zirconocendichlorides isoliert werden. ¹H-NMR (CD₂Cl₂, 200 MHz): δ = 7.4-7.1 (m, 20H, Ph-H), 6.18 (m, 4H, Cp-H), 6.02 (4H, Cp-H), 1.60 (d, 12H, CH₃, ³J_{P-H} = 14.3 Hz) ppm. trans-Dichlorobis{[1-(1-methyl-1-diphenylphosphinoethyl)-η⁵-cyclopentadienyl]dichlorozirconium(IV)-κ²P,P'}palladium(II) 745 mg (1.0 mmol) Bis[1-(1-methyl-1-diphenylphosphinoethyl)-η⁵-cyclopentadienyl]dichlorozirconium(IV) und 384 mg (1.0 mmol) Bis(benzonitril)dichloropalladium(II) werden getrennt in je 30 ml THF gelöst und bei -40 °C gleichzeitig zu 25 ml THF getropft. Die Lösung ist dunkelgelb, beim letzten Tropfen fällt ein kräftig gelber Niederschlag aus, der abfiltriert, zweimal mit je 10 ml THF gewaschen und im Ölpumpenvakuum getrocknet wird.
Ausbeute: 680 mg (0.18 Äq THF) 73 %.
¹H-NMR (CD₂Cl₂, 200 MHz): δ = 7.67 (br, 8H, Ph-H), 7.6-7.2 (m, br, 12H, Ph-H), 6.15 (br s, 4H, Cp-H), 5.97 (br s, 4H, Cp-H), 3.71 (m, br, 4H, THF-H), 1.85 (m, br, 12H, CH₃-H) 1.8 (m, br, 4H, THF-H) ppm.

Beispiel B2: Dichlorobis{[1-(1-methyl-1-diphenylphosphinoethyl)-η⁵-cyclopentadienyl]di-chlorozirconium(IV)-κ²P,P'}platin(II) 75 mg Bis[1-(methyl-1-diphenylphosphinoethyl)-η⁵-cyclopentadienyl]dichlorozirconium(IV) (0.1 mmol) und 47 mg PtCl₂(PhCN)₂ (0.1 mmol) werden in THF zur Reaktion gebracht, nach 2 h ein hellgelber Feststoff isoliert und mit Pentan gewaschen.
Ausbeute: 90 mg (0.09 mmol), 89%.
¹H-NMR (CD₂Cl₂, 200 MHz): δ = 7.7 (m, 8H, Ph-H), 7.4 (m, 12H, Ph-H), 6.12 (m, 4H, Cp-H), 5.91 (m, 4H, Cp-H), 1.85 (pt, 12H, CH₃-H, J = 7.4 Hz) ppm.

Beispiel B3: µ-{Bis[1-(methyl-1-diphenylphosphinoethyl)-η⁵-cyclopentadienyl]dichloro-zirconium(IV)-κP:κP'}-bis[(chlorocyclooctadien)rhodium(l)] 225 mg (0.3 mmol) Bis[1-(methyl-1-diphenylphosphinoethyl)-η⁵-cyclopentadienyl]dichlorozirconium(IV) und 147 mg (0.3 mmol) Cyclooctadien-µ-chlororhodium(l)-Dimer werden getrennt in THF gelöst. Bei -50 °C wird die Zirconocenverbindung zum Rhodiumkomplex getropft und nachfolgend bei RT für 3 h gerührt. Das Lösungsmittel wird im Vakuum entfernt.
¹H-NMR (CD₂Cl₂, 200 MHz): δ = 7.9-7.4 (m, 24H, Ph-H, Cp-H), 6.86 (m, 4H, Cp-H), 5.64 (br, 4H, COD-Vinyl-H), 2.6-2.3 und 2.2-1.6 (m, 20H, COD-Vinyl-und Allyl-H), 1.73 (d, 12H, CH₃, ³J_{P-H} = 13.0 Hz) ppm.

Beispiel B4: Dichlorobis{[1-(1-methyl-1-di-p-tolylphosphinoethyl)-η⁵-cyclopentadienyl]di-chlorozirconium(lV)-κ²P,P'}palladium(II) 1-(1-Methyl-1-ditolylphosphinoethyl)cyclopentadienyllithium 2.65 g Ditolylphosphan (12.4 mmol) werden mit 7.7 ml n-BuLi (12.3 mmol) deprotoniert und nachfolgend 1.6 g 6,6-Dimethylfulven (15.1 mmol) zugesetzt.
Ausbeute: 4.18 g (10.5 mmol), 85 % Mono-THF-Addukt
¹H-NMR (Benzol-d₆, THF-d₈, 200 MHz): δ = 7.50 (pt, 4H, o-Ph-H), 6.96 (d, 4H, m-Ph-H), 6.09 (s, 4H, Cp-H), 3.52 (m, 4H, α-THF-H), 2.08 (s, 6H, CH₃-H), 1.63 (d, 6H, 2'-H, ³J_{P-H} = 12.2 Hz), 1.45 (m, 4H, β-THF-H) ppm.

Bis[1-(1-methyl-1-ditolylphosphinoethyl)-η⁵-cyclopentadienyl]dichlorozirconium 3.65 g (9.15 mmol) 1-(1-Methyl-1-ditolylphosphinoethyl)cyclopentadienyllithium und 1.72 g (4.57 mmol) ZrCl₄ x 2 THF werden jeweils in Toluol suspendiert, bei - 78 °C zusammengegeben, über Nacht auftauen gelassen und aufgearbeitet.
Ausbeute: 2.4 g (68 %) des hellgelben Produktes.
¹H-NMR (CD₂Cl₂, 200 MHz): δ = 7.2-7.0 (m, 16H, Tol-H), 6.18 (m, 4H, Cp-H), 6.01 (4H, Cp-H), 2.34 (s, 12H, Tol-CH₃-H), 1.57 (d, 12H, C2', ³J_{P-H} = 14.3 Hz) ppm.

Dichlorobis{[1 -(1 -methyl-1-di-p-tolylphosphinoethyl)-η⁵-cyclopentadienyl]dichlorozirconium(lV)-κ²P,P'}palladium(II) 47 mg (0.123 mmol) PdCl₂(PhCN)₂ und 98 mg Bis[1-(1-methyl-1-di-*p*-tolylphosphinoethyl)-η⁵-cyclopentadienyl]dichlorozirconium(lV) werden getrennt in Toluol gelöst und gleichzeitig bei -78 °C zu 10 ml Toluol gehebert.
Ausbeute: 100 mg (70%) x 2 Äq. Toluol
¹H-NMR (CD₂Cl₂, 200 MHz): δ = 7.6-7.1 (2 m, 16H, Tol-H), 6.13 (br s, 4H, Cp-H), 5.93 (br s, 4H, Cp-H), 2.40 (s, 12H, Tol-CH₃), 1.78 (pt, br, 12H, CH₃-H, ³⁺⁵J_{P-H} = 8.1 Hz) ppm.

Beispiel B5: Dichlorobis{[1-(1-methyl-1-ditolylphosphinoethyl)-η⁵-cyclopentadienyl]di-chlorozirconium(lV)-κ²P,P'}platin(II) 248 mg Bisphosphan (0.31 mmol) und 146 mg PtCl₂(PhCN)₂ (0.31 mmol) werden in Toluol zur Reaktion gebracht, nach 24 h das Lösungsmittelvolumen um die Hälfte reduziert und nach Zugabe von 20 ml Pentan ein hellgelber Feststoff isoliert. Das Produkt wird mit 2 ml Toluol und dann zweimal mit 5 ml Pentan gewaschen.
Ausbeute: 210 mg (0.20 mmol), 63 %. (C₄₄H₄₈CI₄P₂PtZr, M = 1066.94 g/mol)
¹H-NMR (CD₂Cl₂, 200 MHz): δ = 7.54 (m, 8H, Tol-H), 7.18 (m, 8H, Tol-H), 6.11 (m, 4H, Cp-H), 5.90 (m, 4H, Cp-H), 2.39 (s, 12H, Tol-CH₃), 1.81 (pt, 12H, CH₃-H, J = 8.1 Hz) ppm.

Beispiel B6: Pentachlorotetrahydrofuranopalladium(IV)-chlorobis{[1-(1-tert.butyldiphenyl-phosphinomethyl)-η⁵-cyclopentadienyl]chlorozirconium(lV)-κ²P,P']}µ-chloropalladat(II) 1-(1-tert.Butyldiphenylphosphinomethyl)cyclopentadienyllithium Zu 8.00 ml (8.41g, 45.2 mmol) Diphenylphosphan in 100 ml THF/Pentan werden bei -50 °C 28.5 ml 1.59 M n-BuLi gegeben. Nach 2 h bei Raumtemperatur wird tert.Butylfulven (9.22 g, 55.0 mmol), gelöst in 30 ml Pentan, langsam zugetropft. Nach 2 h wird aufgearbeitet.
Ausbeute: 10.6 g (27.5 mmol) = 61 %.
¹H-NMR (Benzol-d₆, 200 MHz): δ = 7.80-7.63, (m, 4H, o-Ph-H), 7.10-6.90, 6.88-6.80, 6.74-6.66 (m, 6H, Ph-H), 6.25 (br s, 2H, Cp-H), 6.06 (m, 2H, Cp-H), 3.86 (d, 1H, 1'H, ²J_{P-H} = 7.3 Hz), 2.73 (m, 4H, α-THF-H), 1.20 (s, 9H, ^{t}Bu-H), 0.98 (m, 4H, β-THF-H) ppm.

Bis[1-(1-tert.butyldiphenylphosphinomethyl)-η⁵-cyclopentadienyl]dichlorozirconium 3.85 g (9.67 mmol) 1-(1-tert.Butyl-1-diphenylphosphinomethyl)cyclopentadienyllithium x THF in 50 ml Toluol werden bei -78 °C zu einer Suspension von 1.82 g (4.84 mmol) Tetrachlorobis(THF)zirconium(IV) in 50 ml Pentan gegeben und langsam auf Raumtemperatur gebracht, über Nacht gerührt und aufgearbeitet. Die Diastereomerenanreicherung ist abhängig von der Menge des Waschpentans. Im Waschpentan ist das jeweils andere Diastereomer angereichert. Durch Umkristallisation aus CH₂Cl₂/Pentan konnte eine Diastereomerenanreicherung von 5 : 1 erreicht werden.
Ausbeute: 3.16 g (3.95 mmol), 82 %.
¹H-NMR (CD₂Cl₂, 200 MHz): A: δ = 7.6-7.1 (m, 16H, Ph-H), 6.90 (m, 4H, Ph-H), 6.28 (m, 2H, Cp-H), 5.95 (m, 2H, Cp-H), 5.89 (m, 2H, Cp-H), 5.26 (m, 2H, Cp-H), 4.08 (d, 2H, 1'-H, J_{P-H} = 2.9 Hz), 1.26 (s, 18H, ^{t}Bu-H) ppm.
B: δ = 7.6-7.1 (m, 16H, Ph-H), 6.87 (m, 4H, Ph-H), 6.18 (m, 2H, Cp-H), 6.00 (m, 2H, Cp-H), 5.89 (m, 2H, Cp-H), 5.21 (m, 2H, Cp-H), 4.14 (d, 2H, 1'-H, J_{P-H} = 2.7 Hz), 1.27 (s, 18H, ^{t}Bu-H) ppm.

### Pentachlorotetrahydrofuranopalladium(lV)-chlorobis{[1-(1-tert.butyldiphenylphosphinomethyl)-η⁵-cyclopentadienyl]chlorozirconium(lV)-κ²P,P']}µ-chloropalladat(ll)

800 mg Metallocenverbindung (1 mmol) und 384 mg Palladiumverbindung (1 mmol) werden getrennt in je 30 ml THF gelöst und bei -40 °C sehr langsam gleichzeitig zu einer Vorlage von 30 ml THF getropft. Die klare orangefarbene Lösung wird nach 1 Tag zur Trockne gebracht, einige ml THF und 10 ml Pentan zugegeben und ein gelber Feststoff nach 1 h Rühren abfiltriert. Es werden 90 mg des gelben Produktes isoliert. Der Rückstand wird ebenfalls zur Trockne gebracht.
¹H-NMR (CD₂CI₂, 200 MHz): δ = 8.17, 7.74 (je m, je 4H, Ph-H), , 7.55, 7.28 (je m, je 6H, Ph-H), 7.11, 6.90, 6.51, 6.33 (je m, je 2H, Cp-H), 4.62 (pt, 2H, 1'-H, ²⁺⁴J_{P-H} = 5.5 Hz), 4.62 (m, 4H, THF-Pd-H), 3.71 (m, 4H, THF-H), 2.07 (m, 4H, THF-Pd-H), 1.82 (m, 4H, THF-H), 0.63 (s, 18H, ^{t}Bu-H) ppm.
Für die anderen Isomere nur die chem. Verschiebungen der ^{t}Bu-Gruppen: (1.15, 1.13), 0.93, 0.70 ppm.

### Beispiel B7: Bis[carbonylbis{[1-(1-tert.butyldiphenylphosphinomethyl)-η⁵-cyclopenta-dienyl]chloro-µ-chlorozirconium(IV)-κ²P,P']}rhodium(I)]hexachlorozirconium

440 mg (0.55 mmol) Bis[1-(1-tert.butyldiphenylphosphinomethyl)-η⁵-cyclopentadienyl]dichloro-zirconium(IV) und 107 mg (0.275 mmol) Bis[dicarbonyl-µ-chlororhodium] werden bei 0 °C getrennt in Dichlormethan gelöst und zur Lösung des Rhodiumkomplexes getropft. Nach 12 h Rühren wird das Lösungsmittel bis auf 5 ml entfernt und die Fällung mit Pentan vervollständigt. Das Produkt wird auf einer Fritte isoliert und im Ölpumpenvakuum getrocknet.
Ausbeute: 170 mg (1. Fraktion):
¹H-NMR (CD₂Cl₂, 200 MHz): 8.17 (m, 4H, o-Ph-H), 7.6-7.1 (2m, 16H, Ph-H), 6.99, 6.83, 6.26, 6.13 (je m, je 2H, je Cp-H), 4.56 (pt, 2H, 1'-H, J = 5.8 Hz), 0.67 (s, 18H, ^{t}Bu-H) ppm.

### Beispiel B8: µ-{Bis[1-phenyldiphenylphosphinomethyl)-η⁵-cyclopentadienyl]dichlorozirconium(IV)-κP:κP'}-bis[(chlorocyclooctadien)rhodium(I)]

1-(1-Phenyldiphenylphosphinomethyl)cyclopentadienyllithium 2.52 g Diphenylphosphan (13.0 mmol) werden in 25 ml THF gelöst und bei -70 °C 8.15 ml 1.6 M n-Butyllithium zugetropft. Nach 2 h bei RT wird bei -60 °C eine Lösung von 2.02 g (13.1 mmol) 6-Phenylfulven in 30 ml Pentan innerhalb einer Stunde zugetropft, über Nacht gerührt und aufgearbeitet.
Ausbeute : 4.16 g, 80% THF-Addukt (0.75 Äquivalente THF)
¹H-NMR (Benzol-d₆, 200 MHz): δ = 7.57-7.47, 7.44-7.31 (m, 6H, Ph-H), 7.11-6.75 (m, 9H, Ph-H), 6.28 (br s, 2H, Cp-H), 6.12 (m, 2H, Cp-H), 4.89 (d, 1H, ²J_{P-H} = 7.5 Hz, 1'-H) ppm.

Bis[1-{1-phenyldiphenylphosphinomethyl)-η⁵-cyclopentadienyl]dichlorozirconium 1.27 g (3.17 mmol) 1-(1-Phenyl-1-diphenylphosphinomethyl)cyclopentadienyllithium x 0.75 THF und 0.59 g (1.56 mmol) Tetrachlorobis(THF)zirconium(IV) werden gemischt, bei -78 °C Toluol zugesetzt und nach 30 min Rühren bei dieser Temperatur auf Raumtemperatur aufgetaut. Man erhält den Komplex als weißen Feststoff mit einem Diastereomerenverhältnis von 1:1.
Ausbeute: 1.22 g (1.45 mmol), 93 %.
¹H-NMR (CDCl₃, 200 MHz): δ = 7.6-7.0 (m, 60H, Ph-H), 6.08 (m, 2H, Cp-H), 5.98 (m, 2H, Cp-H), 5.90-5.81 (m, 4H, Cp-H), 5.27 (m, 2H, Cp-H), 5.22-5.10 (m, 4H, Cp-H), 5.02 (d, 2H, 1'-H, ²J_{P-H} = 4.2 Hz), 4.91 (d, 2H, 1'-H, ²J_{P-H} = 4.2 Hz), 4.11 (m, 2H, Cp-H) ppm.

µ-{Bis[1-phenyldiphenylphosphinomethyl)-η⁵-cyclopentadienyl]dichlorozirconium(lV)-κP:κP'}-bis[(chlorocyclooctadien)rhodium(l)] 280 mg (0.33 mmol) Bis[1-(phenyldiphenylphosphinomethyl)-η⁵-cyclopentadienyl]dichlorozirconium(IV) und 164 mg (0.33 mmol) µ-Chlorocyclooctadienrhodium(I)-Dimer werden getrennt in THF gelöst. Die Metallocenverbindung wird bei -40 °C tropfenweise zum Rhodiumkomplex gegeben und die gelbe Lösung über Nacht auftauen gelassen. Das Thf wird bis auf 5 ml im Vakuum entfernt und die Fällung durch Pentanzugabe vervollständigt. Isolierung des Produktes auf einer Fritte und zweimaliges Nachwaschen mit Pentan ergibt 150 mg eines gelben Pulvers (2 Diastereomere 1:1).
¹H-NMR (CD₂Cl₂, 200 MHz): (durch die Vielzahl der Signale Überlagerungen weniger intensiver Resonanzen) δ = 8.11, 7.90 (2m), 7.7-6.7 (m, Ph-H), 6.35 (m, 4H, Cp-H), 6.19 (m, 2H, Cp-H), 6.01 (m, 2H, Cp-H), 5.94 (d, 2H, 1'-H, J = 2.2 Hz), 5.88 (d, 2H, 1'-H, J = 2.3 Hz), 5.78 (m, 2H, Cp-H), 5.7-5.4 (v br, 8H, COD-Vinyl-H), 2.9 (br, 8H, COD-Vinyl-H), 2.7-1.7 (sehr breite Signale, 32H, COD-Allyl-H) ppm.

### C: Synthese von weiteren bimetallischen Übergangsmetallverbindungen

### Beispiel C1: Bis[1-(1-methyl-1-diphenylphosphinoethyl)-η⁵-cyclopentadienyl]dichlorotitan

0.52 g (1.74 mmol) und 0.29 g (0.87 mmol) Tetrachlorobis(tetrahydrofuran)titan(IV) werden getrennt in je 15 ml Toluol suspendiert und auf -78 °C abgekühlt. Bei dieser Temperatur wird das Titansalz zum Liganden gegeben und die sich langsam hellbraun färbende Suspension unterhalb von -60 °C gerührt. Nach weiteren 30 min Rühren bei -30 °C ist die jetzt klare Lösung tiefbraunrot. Das Lösungsmittel wird nachfolgend innerhalb von 2 h bei RT im Vakuum entfernt, der Rückstand in 40 ml Dichlormethan aufgenommen und filtriert. Das Filtrat wird zur Trockne gebracht, der Rückstand mit Pentan gewaschen und schließlich im Ölpumpenvakuum getrocknet. Die Ausbeute an noch leicht verunreinigtem Produkt beträgt 0.28 g.
¹H-NMR (CD₂Cl₂, 200 MHz): δ = 7.39 (m, 20H, Ph-H), 6.35 (m, 4H, Cp-H), 6.27 (m, 4H, Cp-H), 1.68 (d, 12H, CH₃, ³J_{P-H} = 15.5 Hz) ppm.

### Beispiel C2: Bis[1-(1-diphenylphosphinoethyl)-η⁵-cyclopentadienyl]dichlorozirconium

1-(1-Diphenylphosphinoethyl)cyclopentadienyllithium 2.58 g Diphenylphosphan (13.9 mmol) in 40 ml THF werden mit 8.6 ml n-BuLi (13.8 mmol) deprotoniert und nachfolgend mit 2.8 g Methylfulven x Pentan (ca. 17 mmol) in 40 ml Pentan innerhalb von 12 h umgesetzt.
Ausbeute: 3.2 g (11.3 mmol), 81 %.
¹H-NMR (Benzol-d₆, THF-d₈, 200 MHz): δ = 7.64 (m, 2H, o-Ph-H), 7.43 (m, 2H, o-Ph-H), 7.12-6.95 (m, 6H, m,p-Ph-H), 6.01 (s, 4H, Cp-H), 3.80 (dq, 1H, 1'-H, ²J_{H-H} = 7.1 Hz, ²J_{P-H} = 7.1 Hz), 1.48 (dd, 3H, CH₃-H, ²J_{H-H} = 7.1 Hz, ²J_{P-H} = 14.7 Hz) ppm.

Bis[1-(1-diphenylphosphinoethyl)-η⁵-cyclopentadienyl]dichlorozirconium 2.72 g (9.58 mmol) Li-Salz in 100 ml Pentan/ Toluol werden zu 1.81 g (4.79 mmol) ZrCl₄ x 2THF in 30 ml Pentan gegeben.
Ausbeute: 2.48 g (3.46 mmol), 72 %.
¹H-NMR (CD₂CI₂, 200 MHz): δ = 7(m, 20H, Ph-H), (m, 4H, Cp-H), (4H, Cp-H), (d, 12H, CH₃, ³J_{P-H} = 14.3 Hz) ppm.

### Beispiel C3: Bis[1-(1-ditolylphosphinoethyl)-η⁵-cyclopentadienyl]dichlorozirconium 1 -(1 -Ditolylphosphinoethyl)cyclopentadienyllithium

1.94 g Ditolylphosphan (9.06 mmol) in 40 ml THF werden mit 5.7 ml n-BuLi (9.1 mmol) deprotoniert und nachfolgend mit 1.1 g Methylfulven x 0.33 Pentan (ca. 11 mmol) in 40 ml Pentan innerhalb von 12 h umgesetzt.
Ausbeute: 2.18 g (6.99 mmol), 77 %.
¹H-NMR (Benzol-d₆, THF-d₈, 200 MHz): δ = 7.63 (pt, 2H, o-Tol-H), 7.50 (pt, 2H, o-Tol-H), 6.99 (pd, 2H, m-Tol-H), 6.91 (pd, 2H, m-Tol-H), 6.01 (s, 4H, Cp-H), 3.90 (dq, 1H, 1'-H, ²J_{H-H} = 7.2 Hz, ²J_{P-H} = 7.2 Hz), 2.08, 2.02 (je s, je 3H, Tol-CH₃), 1.59 (dd, 3H, CH₃-H, ²J_{H-H} = 7.1 Hz, ²J_{P-H} = 14.6 Hz) ppm.

### Bis[1-(1-ditolylphosphinoethyl)-η⁵-cyclopentadienyl]dichlorozirconium

1.95 g (6.25 mmol) Li-Salz in 100 ml Toluol werden zu 1.18 g (3.12 mmol) ZrCl₄ x 2THF in 30 ml Pentan gegeben. Nach Aufarbeitung erhält man einen beigefarbenen Feststoff.
Ausbeute: 810 mg (1.05 mmol), 34 %.
¹H-NMR (CD₂Cl₂, 200 MHz): δ = 7.36 (m, 8H, Tol-H), 7.2-7.0 (m, 24H, Tol-H), 6.3-6.1 (m, 12H, Cp-H), 5.99 (4H, Cp-H), 5.33 (4H, Cp-H), 3.85 (m, 4H, 1'-H), 2.34, 2.31 (je s, je 12H, Tol-CH₃), 1.30 (dd, 6H, CH₃, ³J_{P-H} =14.5 Hz, ³J_{H-H} = 7.1 Hz), 1.28 (dd, 6H, CH₃, ³J_{P-H} =14.6 Hz, ³J_{H-H} = 7.0 Hz) ppm.

### Beispiel C4: Bis[1-(1-tert.butylditolylphosphinomethyl)-η⁵-cyclopentadienyl]dichlorozirconium

1-(1-tert.Butylditolylphosphinomethyl)cyclopentadienyllithium 4.08 g Ditolylphosphan (19.1 mmol) in 50 ml THF werden mit 11.8 ml n-BuLi (18.9 mmol) deprotoniert und nachfolgend 3.45 g 6-tert.Butylfulven x 0.25 Et₂O (ca. 23 mmol) in 40 ml Pentan zugetropft. Nach 12 h wird aufgearbeitet.
Ausbeute: 6.48 g (15.2 mmol), 80 % Mono-THF-Addukt.
¹H-NMR (Benzol-d₆, THF-d₈, 200 MHz): δ = 7.70 (pt, 2H, o-Tol-H, J = 7.8 Hz), 7.61 (pt, 2H, o-Tol-H, J = 7.8 Hz), 6.91 (pd, 2H, m-Tol-H, J = 7.9 Hz), 6.72 (pd, 2H, m-Tol-H, J = 7.8 Hz), 6.22 (br s, 2H, Cp-H), 6.03 (m, 2H, Cp-H), 3.87 (d, 1 H, 1'H, ²J_{P-H} = 7.2 Hz), 3.47 (m, 4H, α-THF-H), 2.01, 1.86 (je s, je Tol-CH₃-H), 1.39 (m, 4H, β-THF-H), 1.20 (s, 9H, ^{t}Bu-H) ppm.

Bis[1-(1-tert.butylditolylphosphinomethyl)-η⁵-cyclopentadienyl]dichlorozirconium 2.26 g (5.31 mmol) 1-(1-tert.Butyl-1-diphenylphosphinomethyl)cyclopentadienyllithium x THF in 40 ml Toluol werden bei -78 °C zu einer Suspension von 1.00 g (2.65 mmol) Tetrachlorobis(THF)zirconium(IV) in 20 ml Pentan gegeben und die Reaktionsmischung über Nacht auf Raumtemperatur aufgetaut. Die Abtrennung von Nebenprodukten erfolgt durch Aufrühren in 50 ml kaltem Heptan, da das Produkt in Pentan zu gut löslich ist und nachfolgende Filtration. Die Diastereomerenanreicherung beträgt etwa 2:1.
Ausbeute: 1.1 g (1.28 mmol), 49 %.
¹H-NMR (CD₂Cl₂, 200 MHz): A: δ = 6.26, 5.99, 5.94, 5.31 (je m, je 2H, Cp-H), 3.95 (d, 2H, 1'-H, J_{P-H} = 2.4 Hz), 1.22 (s, 18H, ^{t}Bu-H) ppm.
B: δ = 6.16, 6.04, 5.94, 5.21 (je m, je 2H, Cp-H), 4.01 (d, 2H, 1'-H, J_{P-H} = 2.2 Hz), 1.23 (s, 18H, ^{t}Bu-H) ppm.
A und B: 7.4-7.2 und 7.0-6.6 (m, 32H, Tol-H), 2.37, 2.36, 2.29, 2.26 (je s, je 6H, Tol-CH₃) ppm.

### Beispiel C5: Bis[1-(1-phenyldiphenylphosphinomethyl)-η⁵-cyclopentadienyl]dichlorotitan(lV)

408 mg (1.0 mmol) 1-(1-Phenyl-1-diphenylphosphinomethyl)cyclopentadienyllithium x 0.75 THF und 166 mg (0.5 mmol) Tetrachlorobis(tetrahydrofuran)titan(IV) werden gemischt, mit 25 ml auf -78 °C vorgekühltem Toluol bei dieser Temperatur versetzt. Nach 36 h Rühren wird aufgearbeitet.
Ausbeute: 0.22 g (0.28 mmol), 55 % im Diastereomerenverhältnis 1:1.
¹H-NMR (CDCl₃, 200 MHz): δ = 7.50-7.05 (m, 60H, Ph-H), 6.27 (m, 2H, Cp-H), 6.09 (m, 2H, Cp-H), 5.98 (m, 4H, Cp-H), 5.38 (m, 4H, Cp-H), 5.19-5.07 (m, 4H, Cp-H und 1'-H), 4.99 (d, 2H, 1'-H, ²J_{P-H} = 3.3 Hz), 3.99 (m, 2H, Cp-H) ppm.

### Beispiel C6: Bis[1-Tolyldiphenylphosphinomethyl)-η⁵-cyclopentadienyl]dichlorozirconium

1-(1-Tolyldiphenylphosphinomethyl)cyclopentadienyllithium 5.6 g (30.1 mmol) Diphenylphosphan werden in 50 ml THF mit 18.8 ml n-Buli (30.1 mmol) deprotoniert. Nach 3 h werden 6.8 g Tolylfulven in 50 ml Pentan zugetropft, die anfallende Suspension über Nacht gerührt und aufgearbeitet.
Ausbeute: 9.0 g (25.0 mmol), 83 %.
¹H-NMR (Benzol-d₆, THF-d₈, 200 MHz): δ = 7.3-6.9 (m, 12H, PPh₂-H, o-Tol-H), 6.86 (d, 2H, m-Tol-H, J = 8.0 Hz), 5.83 (m, 2H, Cp-H), 5.64 (m, 2H, Cp-H), 4.64 (d, 1H, 1'H, ²J_{H-H} ²J_{P-H} = 7.8 Hz), 2.19 (s, 3H, CH₃-H) ppm.

Bis[1-Tolyldiphenylphosphinomethyl)-η⁵-cyclopentadienyl]dichlorozirconium 2.64 g (7.33 mmol) Li-Salz werden in 75 ml Pentan/Toluol 1:1 suspendiert und bei-78 °C zu einer Suspension von 1.36 g (3.61 mmol) Zirconiumtetrachlorid-Bis-THF-Addukt in 30 ml Pentan gegeben und über Nacht gerührt.
Ausbeute: 2.38 g (2.74 mmol), 76 %.
¹H-NMR (CD₂CI₂, 200 MHz): δ = 7.4-7.0 (m, 56H, Ph-H, Tol-H), 6.06 (m, 2H, Cp-H), 5.98 (m, 2H, Cp-H), 5.91-5.85 (m, 4H, Cp-H), 5.21 (m, 4H, Cp-H), 5.08 (m, 2H, Cp-H), 5.01 (d, 2H, 1'-H, ²J_{P-H} = 4.2 Hz), 4.91 (d, 2H, 1'-H, ²J_{P-H} = 4.3 Hz), 4.22 (m, 2H, Cp-H), 2.32, 2.29 (je s, je 6H, CH₃-H) ppm.

### Beispiel C7: Bis[4,7-dimethyl-1-(1-phenyldiphenylphosphinomethyl)-η⁵-indenyl]dichloro-zirconium

4,7-Dimethyl-1-(1-phenyldiphenylphosphinomethyl)indenyllithium Zu einer Lösung von 1.52 g (7.9 mmol) Lithiumdiphenylphosphid in 10 ml THF werden innerhalb von 4 h bei -10 °C 1.83 g (7.9 mmol) E-4,7-Dimethyl-10-phenylbenzofulven, gelöst in 10 ml THF, getropft Lösung 5 Tage bei Raumtemperatur gerührt. Das THF wird im Ölpumpenvakuum entfernt und das Rohprodukt in Pulverform gebracht. Nach Überführung auf eine Fritte und dreimaligem Waschen mit je 10 ml Pentan wird im Ölpumpenvakuum getrocknet. Das isolierte bräunliche pulverförmige Produkt fällt als Bis-THF-Addukt an.
¹H-NMR (Benzol-d₆, 200 MHz): δ = 7.7-7.55, 7.45- 7.3, 7.0-6.7, 6.36 (m, 19H, Ph-H, Ind-H), 5.88 (d, 1H, 1'-H, ²J_{P-H} = 7.0 Hz), 2.99 (s, 3H, CH₃), 2.86 (br, 8H, α-H, THF), 2.56 (s, 3H, CH₃), 1.07 (br, 8H, β-H, THF) ppm.

Bis[4,7-dimethyl-1-(1-phenyldiphenylphosphinomethyl)-η⁵-indenyl]dichlorozirconium 852 mg (1.5 mmol) und 283 mg (0.75 mmol) Tetrachlorobis(tetrahydrofuran)zirconium(IV) werden fest gemischt, bei -78 °C in 20 ml Toluol gelöst und nachfolgend auf RT erwärmt. Nach 1 Tag Rühren wird für eine Stunde auf 70 °C erhitzt und anschließend an der Ölpumpe zur Trockne gebracht. Die nach Zugabe von 30 ml Dichlormethan entstehende Suspension wird filtriert und das Filtrat im Ölpumpenvakuum zur Trockne gebracht. Der Feststoff wird mit Pentan gewaschen und schließlich im Ölpumpenvakuum getrocknet. Es entsteht eine Mischung dreier Diastereomere.
¹H-NMR (Benzol-d₆, 200 MHz): δ = 8.0-6.8 (m, sehr intensiv), 6.83 (d, ²J_{P-H} = 3.9 Hz), 5.76, 5.59 (s oder d), 5.37 (d, ²J_{P-H} = 3.2 Hz), 2.7-2.0 (mehrere s, CH₃), 1.43 (s, 3H,) ppm.

### D: Darstellung von bimetallischen Übergangsmetallverbindungen mit substituierten Dimethylaminomethylen Metallocendichloriden

### Beispiel D1: cis-Dichloro)bis[η⁵-(1-dimethylaminoethyl)cyclopentadienyl]dichlorotitan-κ²P,P']-palladium(ll) 6-(Dimethylamino)6-Methylfulven

Die Umsetzung des aus 47.0 g (0.54 mol) Acetamid und 68.1 g (0.54 mol) Dimethylsulfat gebildeten N,N-Dimethylaminomethoxyethyl-Kations mit Natriumcyclopentadienid ergibt das 6-Dimethylamino-6-methylfulven in einer
Ausbeute von 51.8 g (0.38 mol)
¹H-NMR ([D₁]-Chloroform): δ = 2.51 (s, 3H, C(CH₃); 3.38 (s, 6H, (CH₃)₂N); 6.35, 6.49, 6.65, 6.70 (je m, je 1H, Cp-H).

### 1-Dimethylaminoethyl)cyclopentadienyllithium

Die Umsetzung von 9.33g (77.0 mmol) 6-(Dimethylamino)fulven in 120 ml Diethylether mit 51 ml (77.0 mmol) 1.6 M Methyllithiumlösung in Diethylether ergibt nach Filtration 9.81 g (68.5 mmol) (89%) des Produktes als hellbraunes Pulver.
¹H-NMR ([D₆]-Benzol / [D₈]-Tetrahydrofuran, 5:1): δ = 1.39 (d, ³J = 6.8 Hz, 3H, CH₃); 2.14 (s, 6H, (CH₃)₂N); 3.59 (q, ³J = 6.8 Hz, 1H, CH); 5.74, 5.81 (je m, je 2H, Cp-H).

### Bis[η⁵-(1-dimethylaminoethyl)cyclopentadienyl]dichlorotitan

Eine auf 0 °C gekühlte Lösung von 4.36 g (30.5 mmol) (1-Dimethylaminoethyl)cyclopentadienyllithium 7 in 150 ml Diethylether wird unter Rühren portionsweise mit der ebenfalls auf 0 °C abgekühlten gelben Suspension von 5.08 g (15.3 mmol) TiCl₄·2THF 1 in 40 ml Diethylether versetzt. Anschließend wird bei 0 °C 15 min gerührt, wobei aus der dunkelbraunen eine tiefrote Suspension entsteht. Der Niederschlag wird abfiltriert und dreimal mit je 10 ml Diethylether gewaschen. Der verbleibende rote Niederschlag wird sodann mit soviel Dichlormethan ausgewaschen, bis sich dieses nicht mehr rot färbt. Die tiefrote Dichlormethanphase wird im Ölpumpenvakuum trockengezogen. Man erhält 2.7 g (6.92 mmol) (45%) des Gemisches der beiden Diastereomere und im Verhältnis 1:1 in Form eines rotbraunen Pulvers.
¹H-NMR ([D₂]-Dichlormethan): δ = 1.24, 1.26 (je d, je ³J = 6.6 Hz, je 6H, CH₃); 2.2 (s, 24H, (CH₃)₂N); 3.1 (m, 4H, CH); 6.5 (m, 16H, Cp-H).
cis-Dichloro)bis[η⁵-(1-dimethylaminoethyl)cyclopentadienyl]dichlorotitan-κ²P,P']-palladium(II)
153 mg (0.87 mmol) Bis[η⁵-(1-dimethylaminoethyl)cyclopentadienyl]dichlorotitan und 333 mg (0.87 mmol) Bis(benzonitril)dichloropalladium(II) werden zur Reaktion gebracht. Das rote Produkt wird im Ölpumpenvakuum getrocknet.
Ausbeute: 245 mg (0.696 mmol), 80%.
¹H-NMR (CD₂Cl₂, 200 MHz): δ = 1.23, 1.25 (je d, je ³J = 6.6 Hz, je 6H, CH₃); 2.3 (s, 24H, (CH₃)₂N); 3.2 (m, 4H, CH); 6.4 (m, 16H, Cp-H).

### Beispiel D2: Dichlorobis[η⁵-(1-dimethylaminoethyl)cyclopentadienyl]dichlorozirconium-(lV)-κ²P,P']-platin(II)

Bis[η⁵-(1-dimethylaminoethyl)cyclopentadienyl]dichlorozirconium(lV)
Eine auf 0 °C abgekühlte braune Suspension von 3.25 g (22.6 mmol) (1-Dimethylaminoethyl)cyclopentadienyllithium in 50 ml Diethylether wird portionsweise mit einer ebenfalls auf 0 °C abgekühlten Suspension aus 2.50 g (10.7 mmol) ZrCl₄·2 THF in 20 ml Diethylether versetzt. Anschließend wird 15 min bei 0 °C gerührt, wobei aus der dunklen eine hellbraune Suspension entsteht. Der hellbraune Niederschlag wird abfiltriert und dreimal mit je 10 ml Diethylether gewaschen. Die entstehende hellrote Diethyletherphase enthält das gewünschte Produkt nur stark verunreinigt und wird verworfen. Der verbleibende Rückstand wird solange mit Dichlormethan ausgewaschen, bis sich dieses nicht mehr dunkel färbt. Nach dem Entfernen des Lösungsmittels im Ölpumpenvakuum erhält man 2.03 g (4.67 mmol) (44%) des 1:1-Gemisches der beiden Diastereomere als braunes Pulver.
¹H-NMR ([D₆]-Benzol): δ = 1.24,1.26 (je d, je ³J = 6.8 Hz, 12H, CH₃); 2.0 (s, 24H, (CH₃)₂N); 4.1 (pq, 4H, CH); 5.7-5.9 (m, 8H, Cp-H); 6.1-6.3 (m, 8H, Cp-H).

### Dichlorobis[η⁵-(1-dimethylaminoethyl)cyclopentadienyl]dichlorozirconium(lV)-κ²P,P']-platin(II)

45 mg Bis[η⁵-(1-dimethylaminoethyl)cyclopentadienyl]dichlorozirconium(IV) (0.1 mmol) und 47 mg PtCl₂(PhCN)₂ (0.1 mmol) werden in THF zur Reaktion gebracht, 4 h gerührt und das Produkt nach Pentanzugabe als nahezu unlöslicher Feststoff isoliert.
Ausbeute: 45 mg (0.068 mmol), 68%
¹H-NMR ([D₆]-Benzol): δ = 1.24,1.26 (je d, je ³J = 6.8 Hz, 12H, CH₃); 2.0 (s, 24H, (CH₃)₂N); 4.1 (pq, 4H, CH); 5.7-5.9 (m, 8H, Cp-H); 6.1-6.3 (m, 8H, Cp-H).

### Beispiel D3: Di-µ-chlorobis[bis[η⁵-(1-dimethylaminobenzyl)cyclopentadienyl]dichloro-zirconium]-κ²P,P']-rhodium(I)

(l-Dimethylaminobenzyl)cyclopentadienyllithium
Zu einer braunen Suspension von 3.07 g (25.3 mmol) 6-(Dimethylamino)fulven in 40 ml Diethylether werden bei -40 °C unter Rühren 25 ml (25.3 mmol) 0.99 M Phenyllithiumlösung in Diethylether getropft. Unter langsamen Erwärmen auf Raumtemperatur wird über Nacht gerührt. Es entsteht eine dunkelviolette Suspension. Nach dem Entfernen des Lösungsmittels im Vakuum bleibt ein klebriger violetter Feststoff zurück. Durch Zugabe von 20 ml Pentan und 12 h Rühren entsteht eine helle Suspension. Nach dem Absitzen wird die Pentanphase abgehebert und der Rückstand im Ölpumpenvakuum getrocknet. Man erhält 5.0 g (24.6 mmol) (97%) Produkt als helles, leicht violettes Pulver.
¹H-NMR ([D₆]-Benzol / [D₈]-Tetrahydrofuran, 3:1) δ = 2.34 (s, 6H, (CH₃)₂N); 4.20 (s, 1H, CH); 6.09 (ps, 4H, Cp-H); 6.96 (m, 1H, Ph-H); 7.12 (m, 2H, Ph-H); 7.69 (m, 2H, Ph-H).

### Bis[η⁵-(1-dimethylaminobenzyl)cyclopentadienyl]dichlorozirconium

Es werden ein Gemisch aus 401 mg (2.0 mmol) (1-Dimethylaminobenzyl)cyclopentadienyllithium und 233 mg (1.0 mmol) ZrCl₄ in 50 ml Tetrahydrofuran bei -78°C aufgenommen. Anschließend wird 15 min bei 0 °C gerührt, wobei aus der dunklen eine hellbraune Suspension entsteht. Der hellbraune Niederschlag wird abfiltriert und dreimal mit je 10 ml Diethylether gewaschen. Die entstehende hellrote Diethyletherphase enthält das gewünschte Produkt nur stark verunreinigt und wird verworfen. Der verbleibende Rückstand wird solange mit Dichlormethan ausgewaschen, bis sich dieses nicht mehr dunkel färbt. Nach dem Entfemen des Lösungsmittels im Ölpumpenvakuum erhält man 278 mg (0.52 mmol) (52%) des 1:1-Gemisches der beiden Diastereomere als braunes Pulver.
¹H-NMR ([D₈]-Tetrahydrofuran): δ = 2.03, 2.07 (je s, je 12H, (CH₃)₂N); 4.78, 4.85 (je s, je 2H, CH); 5.93, 6.05, 6.13, 6.20, 6.28, 6.63 (je m, 16H, Cp-H); 7.0-7.5 (br, 20H, Ph-H).
Di-µ-chlorobis[bis[η⁵-(1-dimethylaminobenzyl)cyclopentadienyl]dichlorozirconium]-κ²P,P')-rhodium(I)
Bei -30 °C werden 353 mg (0.66 mmol) Bis[η⁵-(1-dimethylaminobenzyl)cyclopentadienyl]dichlorozirconium (IV) in 30 ml CH₂Cl₂ zu 164 mg (0.33 mmol) Cyclooctadien-µ-chlororhodium(l)-Dimer in 20 ml CH₂Cl₂ getropft. Die klare Lösung färbt sich rot. Nach 6 h wird das Dichlormethan bis auf 5 ml entfernt und 30 ml Pentan zugegeben. Das orangegelbe Produkt wird dreimal mit je 5 ml Pentan gewaschen und im Ölpumpenvakuum getrocknet.
Ausbeute: 404 mg (90 %, 0.30 mmol)
¹H-NMR ([D₈]-Tetrahydrofuran): δ = 2.05, 2.07 (je s, je 12H, (CH₃)₂N); 4.82, 4.86 (je s, je 2H, CH); 5.95, 6.04, 6.15, 6.20, 6.29, 6.61 (je m, 16H, Cp-H); 7.0-7.5 (br, 20H, Ph-H).

### Beispiel D4: Carbonylchlorobis[η⁵-(1-dimethylamino-1-methylethyl)-cyclopentadienyl]-chloro-µ-chlorotitanium(lV)-κ²P,P']rhodium(I) (1-Dimethylaminol-methylethyl)cyclopentadienyllithium

Die Umsetzung von 7.72g (57.2 mmol) 6-(Dimethylamino)6-methylfulven mit 33.2 ml (57.2 mmol) 1.72 M Methyllithiumlösung in 120 ml Diethylether ergibt 7.3 g (46.9 mmol) (82%) Produkt als hellbraunes Pulvers.
¹H-NMR ([D₆]-Benzol / [D₈]-Tetrahydrofuran, 10:1) δ = 1.53 (s, 6H, CH₃); 2.19 (s, 6H, (CH₃)₂N); 5.94, 6.00 (je m, je 2H, Cp-H).

### Bis[η⁵-(1-dimethylaminol-methylethyl)cyclopentadienyl]dichlorotitan

Eine auf 0 °C gekühlte Suspension von 1.88 g (11.9 mmol) (1-Dimethylamino1methylethyl)-cyclopentadienyllithium in 50 ml Diethylether wird unter Rühren portionsweise mit einer ebenfalls auf 0 °C abgekühlten gelben Suspension von 2.0 g (11.9 mmol) TiCl₄·2THF in 25 ml Diethylether versetzt. Anschließend wird bei 0 °C 25 min gerührt, wobei aus der dunkelbraunen eine tiefrote Suspension entsteht. Der Niederschlag wird abfiltriert und dreimal mit je 10 ml Diethylether gewaschen. Der verbleibende rote Niederschlag wird sodann mit soviel Dichlormethan ausgewaschen, bis sich dieses nicht mehr rot färbt. Die tiefrote Dichlormethanphase wird im Ölpumpenvakuum zur Trockene gebracht. Man erhält 2.59 g (6.19 mmol) (52%) des Produkts in Form eines rotbraunen Pulvers. Die Etherphase wird um ein Drittel eingeengt und über Nacht bei -30 °C gelagert. Der ausgefallene Niederschlag wird abfiltriert und mit wenig Diethylether gewaschen. Man erhält weitere 0.99g (20%)Produkt.
¹H-NMR ([D₂]-Dichlormethan): δ = 1.53 (s, 6H, CH₃); 2.05 (s, 12H, (CH₃)₂N); 6.50, 6.62 (je m, je 4H, Cp-H).

### Carbonylchlorobis[η⁵-(1-dimethylamino-1-methylethyl)cyclopentadienyl]chloro-µ-chlorotitanium(lV)-κ²P,P']rhodium(I)

213 mg Bis[η⁵-(1-dimethylaminol-methylethyl)cyclopentadienyl]dichlorotitan (0.51 mmol) und 100 mg Bis[dicarbonyl-µ-chlororhodium] (0.254 mmol) werden getrennt in je 30 ml CH₂Cl₂ gelöst. Bei 0 °C wird die Titaniumverbindung zum Rhodiumkomplex getropft und die Reaktionsmischung 24 h bei Raumtemperatur gerührt. Das Dichlormethan wird bis auf 5 ml im Vakuum entfernt und die Fällung mit 20 ml Pentan vervollständigt. Das rote Produkt wird auf einer Fritte isoliert und im Ölpumpenvakuum getrocknet.
Ausbeute: 265 mg (0.45 mmol), 89 %.
¹H-NMR ([D₂]-Dichlormethan): δ = 1.55 (s, 6H, CH₃); 2.15 (s, 12H, (CH₃)₂N); 6.53, 6.66 (je m, je 4H, Cp-H).

### Beispiel D5: Dichlorobis[η⁵-(1-dimethylaminoethyl)cyclopentadienyl]dichlorozirconium-(lV)-κ²P,P']-nickel(II)

Bis[η⁵-(1-dimethylamino1-methylethyl)cyclopentadienyl]dichlorozirconium Eine auf 0 °C abgekühlte gelbbraune Suspension von 5.71 g (36.1 mmol) (1-Dimethyl-amino1-methylethyl)cyclopentadienyllithium in 250 ml Diethylether wird portionsweise mit einer ebenfalls auf 0 °C abgekühlten Suspension aus 6.82 g (18.1 mmol) ZrCl₄·2 THF in 60 ml Diethylether versetzt. Anschließend wird 15 min bei 0 °C gerührt, wobei aus der gelbbraunen eine gelbliche Suspension entsteht. Der gelbbeige Niederschlag wird abfiltriert und dreimal mit je 20 ml Diethylether gewaschen. Die Diethyletherphase wird im Ölpumpenvakuum bis zur Hälfte eingeengt und bei - 30 °C gelagert. Der verbleibende Rückstand wird solange mit Dichlormethan ausgewaschen, bis sich dieses nicht mehr dunkel färbt. Nach dem Entfernen des Lösungsmittels im Ölpumpenvakuum erhält man 4.44 g (9.6 mmol) (53 %) des Produkts als hellgelbes Pulver. Ein über Nacht aus der Diethyletherphase ausgefallener Niederschlag wird abfiltriert und im Vakuum getrocknet. Man erhält weitere 1.51 g ( 3.3 mmol) (18 %) Produkt.
¹H-NMR ([D₂]-Dichlormethan): δ = 1.51 (s, 6H, C(CH₃)₂); 1.96 (s, 6H, N(CH₃)₂); 6.37, 6.47 (je m, je 4H, Cp-H).

### Dichlorobis[η⁵-(1-dimethylaminoethyl)cyclopentadienyl]dichlorozirconium(IV)-κ²P,P']-nickel(II)

440 mg Bis[η⁵-(1-dimethylaminoethyl)cyclopentadienyl]dichlorozirconium(IV) (1.0 mmol) und 335 mg NiCl₂(PhCN)₂ (1.0 mmol) werden in THF zur Reaktion gebracht, 4 h gerührt und das Produkt nach Pentanzugabe als nahezu unlöslicher Feststoff isoliert.
Ausbeute: 349 mg (0.062 mmol), 62%
¹H-NMR ([D₂]-Dichlormethan): δ = 1.52 (s, 6H, C(CH₃)₂); 1.96 (s, 6H, N(CH₃)₂); 6.38, 6.48 (je m, je 4H, Cp-H).

### Beispiel D6: [η⁵-(1-Dimethylamino-1-methylbenzyl)cyclopentadienyl][η⁵-cyclopentadienyl]di-chlorozirconium

Zu einer auf - 20 °C abgekühlten Suspension von 2.03 g (5.0 mmol) (η⁵-cyclopentadienyl)trichlorozirconium in 50 ml Diethylether wird bei dieser Temperatur die braune Lösung von 0.78 g (5 mmol) (1-Dimethylamino1-methylethyl)-cyclopentadienyllithium in 50 ml eines 1:1 Gemisches aus Tetrahydrofuran und Diethylether innerhalb von 1 h zugetropft. Die Suspension färbt sich gelbbräunlich. Anschließend wird das entstandene Lithiumchlorid über Cellite abfiltriert. Die anfangs leicht trübe, später klargelbe Lösung wird zur Trockene gebracht. Der beigegelbe Rückstand wird mit 100 ml Pentan versetzt und über Nacht gerührt. Der verbleibende Rückstand wird abfiltriert und im Ölpumpenvakuum getrocknet. Man erhält 1.01 g (2.6 mmol) (52 %) vom Produkt in Form eines hellen Pulvers. ¹H-NMR ([D₂]-Dichlormethan): δ = 1.53 (s, 6H, CH₃); 2.02 (s, 12H, N(CH₃)₂); 6.39, 6.50 (je m, je 2H, Cp'-H); 6.49 (s, 5H, Cp-H).
E: Beispiele zur Hydroformylierung von Olefinen

### Typische Reaktionsführung:

0.08 mmol der jeweiligen bimetallischen Übergangsmetallverbindung werden in 25 ml Toluol oder THF gelöst und es werden 8 ml 1-Hexen (64 mmol) zugegeben. Das Verhältnis Rh : Alken beträgt 1 : 800. Der CO/H₂-Druck beträgt zwischen 20-30 bar (1 : 1) und die Temperatur liegt zwischen 40 und 80°C. Es wird jeweils 1 eq. Triethylamin zugegeben (Verhältnis Rh : Et₃N beträgt 1 : 1).
Ein 150 ml Autoklav wird auf die gewünschte Temperatur gebracht und mit einer zuvor hergestellten Lösung der bimetallischen Übergangsmetallverbindung und des Olefins beschickt. Das Formiergas (CO/H₂) wird aufgepresst und die Reaktion nach 30 min abgebrochen.

| Katalysator | Lösungsmittel | Temp. [°C] | Druck [bar] | n/iso | TOF [mol Aldehyd(mol RH*h] |
|---|---|---|---|---|---|
| A3 | Toluol | 80 | 30 | 5:1 | 130 |
| A4 | Toluol | 80 | 20 | 4:1 | 350 |
| A5 | Toluol | 80 | 20 | 2:1 | 20 |
| A6 | Toluol | 80 | 10 | 1.5:1 | 30 |
| B3 | THF | 80 | 20 | 6:1 | 90 |
| B6 | THF | 60 | 30 | 3:1 | 120 |
| B7 | THF | 55 | 20 | 3:1 | 220 |
| B8 | THF | 60 | 30 | 7:1 | 250 |
| D4 | THF | 60 | 30 | 3:1 | 60 |

## Patentansprüche

1. Katalysatorsystem, enthaltend a) mindestens einen Cokatalysator und b) mindestens eine bimetallische Übergangsmetallverbindung der Formel I 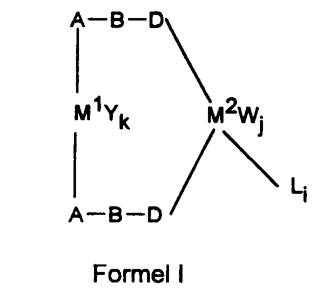 worin
M¹ ein Übergangsmetall der Gruppe III b, IV b, Vb oder VI b des Periodensystems der Elemente ist,
Y entweder gleich oder verschieden ist und ein Wasserstoffatom, eine C₁-C₄₀-kohlenwasserstoffhaltige Gruppe, eine OH-Gruppe, ein Halogenatom, eine NR¹₂-Gruppe, worin R¹ ein Halogenatom, eine C₁-C₁₀-Alkylgruppe oder eine C₆-C₁₀-Arylgruppe ist, bedeutet oder
Y eine substituierte oder unsubstituierte Butadieneinheit ist,
k eine ganze Zahl ist, die der Wertigkeit des Übergangsmetallatoms M¹ minus zwei entspricht und im Fall, wenn Y gleich Butadien bedeutet gleich 1 ist,
A gleich oder verschieden ein substituierter oder unsubstituierter Cyclopentadienylligand ist, wobei mindestens einer substituiert sein muß,
B gleich oder verschieden ist und 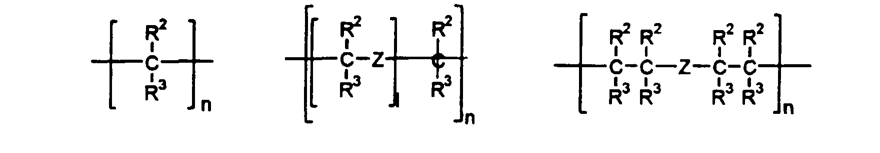 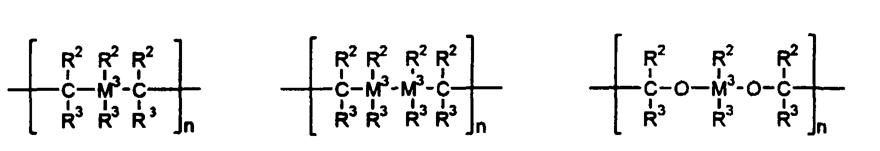 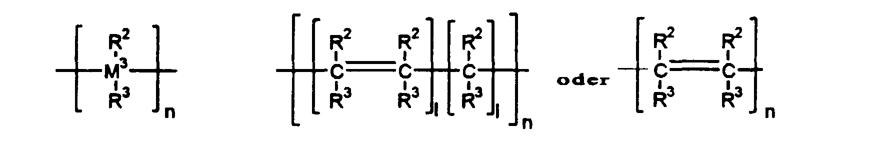 bedeuten, wobei n eine ganze Zahl von 0 bis 20 ist, l eine ganze Zahl von 1 bis 20 ist, Z gleich
〉NR⁴, 〉CO, 〉PR⁴, 〉P(O)R⁴, 〉SO,
SO₂, O oder S ist, worin R⁴ ein Halogenatom, eine C₁-C₁₀-Alkylgruppe oder eine C₆-C₁₀-Arylgruppe ist,
R² und R³ gleich oder verschieden sind und ein Wasserstoffatom, ein Halogenatom, eine C₁-C₄₀-kohlenwasserstoffhaltige Gruppe bedeuten, oder jeweils zwei Reste R², jeweils zwei Reste R³, oder je ein Rest R² und R³ jeweils mit den sie verbindenen Atomen einen oder mehrere Ringe bilden und M³ Silizium, Germanium oder Zinn ist,
D gleich oder verschieden ist und
X-R⁵ ₘ
bedeutet und X an B kovalent und an M² koordinativ gebunden ist, wobei X gleich ein Element der Gruppe Va oder Vla des Periodensystems der Elemente bedeutet und R⁵ gleich oder verschieden ein Wasserstoffatom, ein Halogenatom, eine C₁-C₄₀-kohlenwasserstoffhaltige Gruppe bedeutet, oder jeweils zwei Reste R⁵ mit den sie verbindenen Atomen einen oder mehrere Ringe bilden und m gleich 1 oder 2 ist,
M² ein Metall der Gruppe VI b, VII b, VIII b, IX b oder X b des Periodensystems der Elemente ist,
W entweder gleich oder verschieden ist und ein Wasserstoffatom, eine C₁-C₄₀-kohlenwasserstoffhaltige Gruppe, ein Halogenatom, eine OR¹-Gruppe, eine NR¹₂-Gruppe, worin R¹ ein Wasserstoffatom, ein Halogenatom, eine C₁-C₁₀-Alkylgruppe oder eine C₆-C₁₀-Arylgruppe ist, bedeutet oder W eine substituierte oder unsubstituierte Butadieneinheit ist,
j eine ganze Zahl 0, 1, 2, 3, 4, 5 oder 6 ist, die der Wertigkeit des Übergangsmetallatoms M² entspricht,
L einen neutralen Donorliganden bedeutet, und
i eine ganze Zahl von 0 bis 10 ist.

2. Katalysatorsystem gemäß Anspruch 1, worin die bimetallische Übergangsmetallverbindung die Formel (II) aufweist 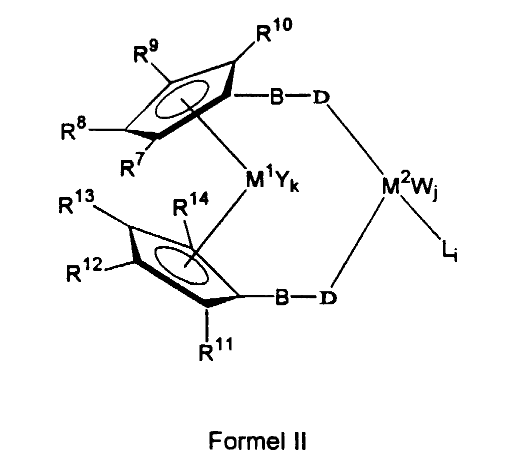 worin
M¹ ein Übergangsmetall der Gruppe III b, IV b, V b oder VI b des Periodensystems der Elemente ist,
Y bevorzugt gleich oder verschieden ist und ein Wasserstoffatom, eine C₁-C₂₀-kohlenwasserstoffhaltige Gruppe ist,
k eine ganze Zahl ist, die der Wertigkeit des Übergangsmetallatoms M¹ minus zwei entspricht,
R⁷, R⁸, R⁹, R¹⁰, R¹¹, R¹², R¹³ und R¹⁴ gleich oder verschieden sind und ein Wasserstoffatom, ein Halogenatom, eine C₁-C₄₀-kohlenwasserstoffhaltige Gruppe einen -SiR¹₃, -NR¹₂, -SiOR¹₃, -SiSR¹₃ oder -PR¹₂-Rest bedeuten, worin R¹ ein Halogenatom, eine C₁-C₁₀ Alkylgruppe oder eine C₆-C₁₀-Arylgruppe ist, oder zwei oder mehr benachbarte Reste R⁷, R⁸, R⁹ und R¹⁰ oder R¹¹, R¹², R¹³ und R¹⁴ zusammen mit den sie verbindenen Atomen ein Ringsystem bilden, oder ein Rest R⁷, R⁸, R⁹ oder R¹⁰ mit einem Rest R¹¹, R¹², R¹³ oder R¹⁴ eine Verbrückung zwischen den beiden Cyclopentadienylringen darstellt,
B gleich 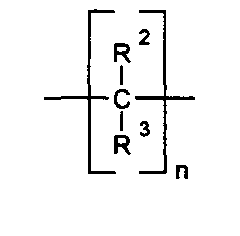 ist, wobei n eine ganze Zahl von 1 bis 8 insbesondere 1, 2, 3 oder 4 ist und R² und R³ gleich oder verschieden sind und ein Wasserstoffatom, ein Halogenatom, eine C₁-C₂₀-kohlenwasserstoffhaltige Gruppe bedeuten, oder jeweils zwei Reste R², jeweils zwei Reste R³, oder R² und R³ jeweils mit den sie verbindenen Atomen einen oder mehrere Ringe bilden,
D gleich
X-R⁵ ₘ
bedeutet, wobei X gleich ein Element der Gruppe Va des Periodensystems der Elemente, wie Stickstoff oder Phosphor, oder der Gruppe VI a des Periodensystems der Elemente, wie Sauerstoff oder Schwefel, bedeutet und R⁵ gleich oder verschieden ist und ein Wasserstoffatom oder eine C₁-C₁₀-kohlenwasserstoffhaltige Gruppe bedeutet, oder jeweils zwei Reste R⁵ mit den sie verbindenen Atomen einen oder mehrere Ringe bilden,
sowie im Fall, wenn X gleich Stickstoff oder Phosphor bedeutet m gleich 2 ist, und im Fall, wenn X gleich Sauerstoff oder Schwefel ist m gleich 1 ist, M² ein Metall der Gruppe VIII b des Periodensystems der Elemente wie Eisen, Ruthenium, Osmium, Cobalt, Rhodium, Iridium, Nickel, Palladium oder Platin ist,
W bevorzugt gleich oder verschieden ist und ein Wasserstoffatom, eine C₁-C₂₀-kohlenwasserstoffhaltige Gruppe, ein Halogenatom, eine NR¹₂-Gruppe, worin R¹ ein Halogenatom, eine C₁-C₁₀ Alkylgruppe oder eine C₆-C₁₀-Arylgruppe ist, bedeutet,
j eine ganze Zahl ist, die der Wertigkeit des Übergangsmetallatoms M² entspricht,
L einen neutralen Donorliganden bedeutet
und i gleich 1, 2, 3, 4, 5 oder 6 ist.

3. Katalysatorsystem gemäß Anspruch 2, worin M¹ ein Übergangsmetall der Gruppe IV b des Periodensystems der Elemente ist,
Y bevorzugt gleich oder verschieden ist und eine C₁-C₁₀-kohlenwasserstoffhaltige Gruppe oder ein Halogenatom ist und k eine ganze Zahl ist, die der Wertigkeit des Übergangsmetallatoms M¹ minus zwei entspricht, R⁷, R⁸, R⁹, R¹⁰, R¹¹, R¹², R¹³ und R¹⁴ gleich oder verschieden sind und ein Wasserstoffatom, eine C₁-C₁₀-kohlenwasserstoffhaltige Gruppe oder zwei oder mehr benachbarte Reste R⁷, R⁸, R⁹ oder R¹⁰ oder R¹¹, R¹², R¹³ oder R¹⁴ zusammen mit den sie verbindenen Atomen ein Ringsystem bilden,
B gleich 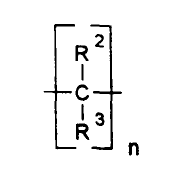 ist, wobei n eine ganze Zahl 1, 2, 3 oder 4 ist und R² und R³ gleich sind und ein Wasserstoffatom, eine C₁-C₁₀-kohlenwasserstoffhaltige Gruppe bedeuten oder R² und R³ jeweils mit den sie verbindenen Atomen einen oder mehrere Ringe bilden,
D gleich
X-R⁵ ₂
bedeutet, wobei X gleich ein Element der Gruppe Va des Periodensystems der Elemente, inbesondere Stickstoff oder Phosphor, bedeutet und R⁵ gleich oder verschieden ist und ein Wasserstoffatom, eine C₁-C₁₀-kohlenwasserstoffhaltige Gruppe bedeutet oder jeweils zwei Reste R⁵ mit den sie verbindenen Atomen einen oder mehrere Ringe bilden,
M² bevorzugt ein Metall der Gruppe VIII b des Periodensystems der Elemente, insbesondere bevorzugt Ruthenium, Cobalt, Rhodium, Nickel oder Palladium ist,
W bevorzugt gleich ist und ein Wasserstoffatom, eine C₁-C₁₀-kohlenwasserstoffhaltige Gruppe oder ein Halogenatom, insbesondere Brom, Chlor, Fluor oder eine NR¹₂-Gruppe, worin R1 ein Halogenatom, eine C₁-C₁₀ Alkylgruppe oder eine C₆-C₁₀-Arylgruppe ist, bedeutet und j eine ganze Zahl ist, die der Wertigkeit des Übergangsmetallatoms M² entspricht,
L einen neutralen Donorliganden bedeutet und
i gleich 1,2,3 oder 4 ist.

4. Katalysator gemäß einem oder mehreren der Ansprüche 1 bis 3, worin der Cokatalysator eine Aluminiumverbindung und/oder eine Borverbindung ist.

5. Verfahren zur Herstellung eines Polyolefins durch Polymerisation eines oder mehrerer Olefine in Gegenwart des Katalysatorsystems gemäß einem oder mehreren der Ansprüche 1 bis 4.

6. Verfahren zur Hydroformylierung eines Olefins mit Kohlenmonoxid und Wasserstoff in Gegenwart einer bimetallischen Übergangsmetallverbindung der Formel I, die wie in Anspruch 1 definiert ist.

7. Bimetallische Übergangsmetallverbindung der (Formel I) 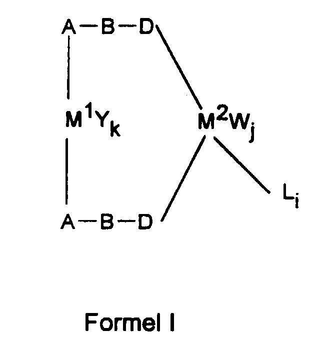 worin
M¹ ein Übergangsmetall der Gruppe III b, IV b Vb, oder VI b des Periodensystems der Elemente ist,
Y entweder gleich oder verschieden ist und ein Wasserstoffatom, eine C₁-C₄₀-kohlenwasserstoffhaltige Gruppe, eine OH-Gruppe, ein Halogenatom, eine NR¹₂-Gruppe, worin R¹ ein Halogenatom, eine C₁-C₁₀-Alkylgruppe oder eine C₆-C₁₀-Arylgruppe ist, bedeutet oder
Y eine substituierte oder unsubstituierte Butadieneinheit ist,
k eine ganze Zahl ist, die der Wertigkeit des Übergangsmetallatoms M¹ minus zwei entspricht und im Fall, wenn Y gleich Butadien bedeutet gleich 1 ist,
A gleich oder verschieden ein substituierter oder unsubstituierter Cyclopentadienylligand ist, wobei mindestens einer substituiert sein muß
B gleich oder verschieden ist und 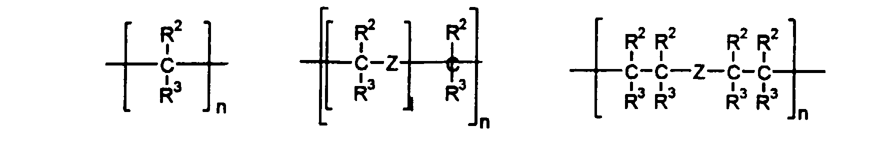 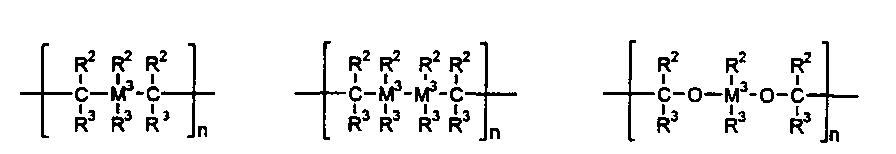 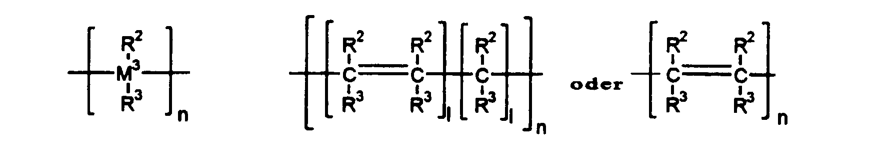 bedeutet, wobei n eine ganze Zahl von 0 bis 20 ist, I eine ganze Zahl von 1 bis 20 ist, Z gleich
〉NR⁴, 〉CO, 〉PR⁴, 〉P(O)R⁴, 〉SO,
SO₂, O oder S ist, worin R⁴ ein Halogenatom, eine C₁-C₁₀-Alkylgruppe oder eine C₆-C₁₀-Arylgruppe ist,
R² und R³ gleich oder verschieden sind und ein Wasserstoffatom, ein Halogenatom, eine C₁-C₄₀-kohlenwasserstoffhaltige Gruppe wie eine C₁-C₂₀-Alkyl-, eine C₁-C₂₀-Alkoxy-, eine C₆-C₂₀-Aryl-, eine C₂-C₂₀-Alkenyl-, eine C₇-C₄₀-Alkylaryl- oder eine C₈-C₄₀-Arylalkenylgruppe bedeuten, oder jeweils zwei Reste R², jeweils zwei Reste R³, oder je ein Rest R² und R³ jeweils mit den sie verbindenen Atomen einen oder mehrere Ringe bilden und M³ Silizium, Germanium oder Zinn ist,
D gleich oder verschieden ist und
X-R⁵ ₘ
bedeutet und X an B kovalent und an M² koordinativ gebunden ist, wobei X gleich Stickstoff ist und R⁵ gleich oder verschieden ein Wasserstoffatom, ein Halogenatom oder eine C₁-C₄₀-kohlenwasserstoffhaltige Gruppe bedeutet, oder jeweils zwei Reste R⁵ mit den sie verbindenen Atomen einen oder mehrere Ringe bilden und m gleich 2 ist,
M² ein Metall der Gruppe VI b, VII b, VIII b, IX b oder X b des Periodensystems der Elemente ist,
W entweder gleich oder verschieden ist und ein Wasserstoffatom, eine C₁-C₄₀-kohlenwasserstoffhaltige Gruppe, ein Halogenatom, eine OR¹-Gruppe, eine NR¹₂-Gruppe, worin R¹ ein Wasserstoffatom, ein Halogenatom, eine C₁-C₁₀ Alkylgruppe oder eine C₆-C₁₀-Arylgruppe ist, bedeutet oder W eine substituierte oder unsubstituierte Butadieneinheit ist,
j eine ganze Zahl 0, 1, 2, 3, 4, 5 oder 6 ist, die der Wertigkeit des Übergangsmetallatoms M² entspricht
L einen neutralen Donorliganden bedeutet und
i eine ganze Zahl von 0 bis 10 ist.

## Claims

1. A catalyst system comprising a) at least one cocatalyst and b) at least one bimetallic transition metal compound of the formula I 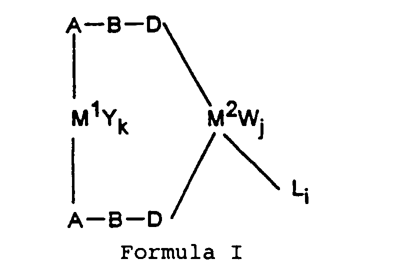 where
M¹ is a transition metal of group IIIb, IVb, Vb or VIb of the Periodic Table of the Elements,
Y is either identical or different and is a hydrogen atom, a C₁-C₄₀-hydrocarbon group, an OH group, a halogen atom, an NR¹₂ group, where R¹ is a halogen atom, a C₁-C₁₀-alkyl group or a C₆-C₁₀-aryl group, or
Y is a substituted or unsubstituted butadiene unit,
k is an integer which corresponds to the valence of the transition metal atom M¹ minus two and, when Y is butadiene, is equal to 1,
A are identical or different and are each a substituted or unsubstituted cyclopentadienyl ligand, at least one of which has to be substituted,
B are identical or different and are 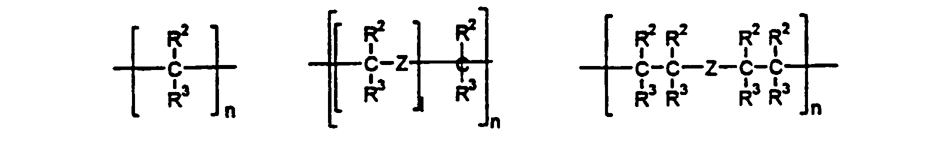 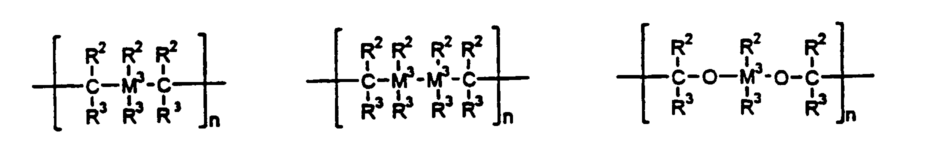 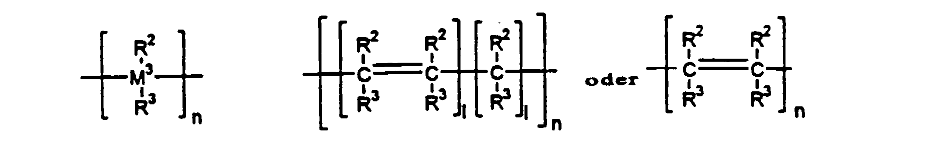 where n is an integer from 0 to 20, l is an integer from 1 to 20, Z is
〉NR⁴, 〉CO, 〉PR⁴, 〉P(O)R⁴, 〉SO,
SO₂, O or S, where R⁴ is a halogen atom, a C₁-C₁₀-alkyl group or a C₆-C₁₀-aryl group,
R² and R³ are identical or different and are each a hydrogen atom, a halogen atom, a C₁-C₄₀-hydrocarbon group, or two radicals R², two radicals R³ or one radical R² and one radical R³, in each case together with the atoms connecting them, form one or more rings, and M³ is silicon, germanium or tin, D are identical or different and are
X-R⁵ ₘ
and X is covalently bonded to B and coordinatively bonded to M², where X is an element of group Va or VIa of the Periodic Table of the Elements and R⁵ are identical or different and are each a hydrogen atom, a halogen atom, a C₁-C₄₀-hydrocarbon group, or two radicals R⁵ together with the atoms connecting them form one or more rings and m is 1 or 2,
M² is a metal of group VIb, VIIb, VIIIb, IXb or Xb of the Periodic Table of the Elements,
W are either identical or different and are each a hydrogen atom, a C₁-C₄₀-hydrocarbon group, a halogen atom, an OR¹ group, an NR¹₂ group, where R¹ is a hydrogen atom, a halogen atom, a C₁-C₁₀-alkyl group or a C₆-C₁₀-aryl group, or W is a substituted or unsubstituted butadiene unit,
j is an integer 0, 1, 2, 3, 4, 5 or 6 which corresponds to the valence of the transition metal atom M²,
L is an uncharged donor ligand and
i is an integer from 0 to 10.

2. A catalyst system as claimed in claim 1, wherein the bimetallic transition metal compound has the formula (II) 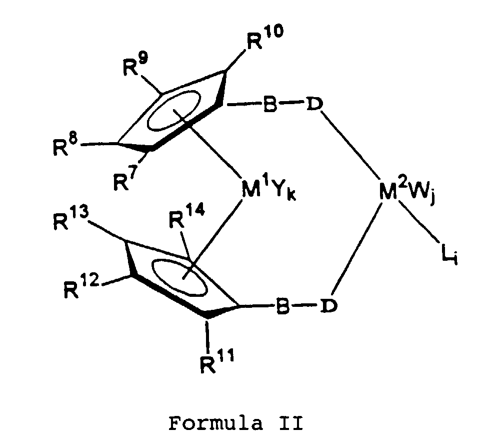 where
M¹ is a transition metal of group IIIb, IVb, Vb or VIb of the Periodic Table of the Elements,
Y are preferably identical or different and are each a hydrogen atom, a C₁-C₂₀-hydrocarbon group,
k is an integer which corresponds to the valence of the transition metal atom M¹ minus two,
R⁷, R⁸, R⁹, R¹⁰, R¹¹, R¹², R¹³ and R¹⁴ are identical or different and are each a hydrogen atom, a halogen atom, a C₁-C₄₀-hydrocarbon group or a -SiR¹₃, -NR¹₂, -SiOR¹₃, -SiSR¹₃ or -PR¹₂ radical, where R¹ is a halogen atom, a C₁-C₁₀-alkyl group or a C₆-C₁₀-aryl group, or two or more adjacent radicals R⁷, R⁸, R⁹ and R¹⁰ or R¹¹, R¹², R¹³ and R¹⁴ together with the atoms connecting them form a ring system, or a radical R⁷, R⁸, R⁹ or R¹⁰ together with a radical R^{11,} R¹², R¹³ or R¹⁴ forms a bridge between the two cyclopentadienyl rings,
B is 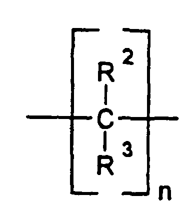 where n is an integer from 1 to 8, in particular 1, 2, 3 or 4, and R² and R³ are identical or different and are each a hydrogen atom, a halogen atom, a C₁-C₂₀-hydrocarbon group, or two radicals R², two radicals R³ or R² and R³, in each case together with the atoms connecting them, form one or more rings,
D is
X-R⁵ _{m,}
where X is an element of group Va of the Periodic Table of the Elements, e.g. nitrogn or phosphorus, or of group VIa of the Periodic Table of the Elements, e.g. oxygen or sulfur, and
R⁵ are identical or different and are each a hydrogen atom or a C₁-C₁₀-hydrocarbon group, or two radicals R⁵ together with the atoms connecting them form one or more rings, and m is 2 when X is nitrogen or phosphorus and m is 1 when X is oxygen or sulfur,
M² is a metal of group VIIIb of the Periodic Table of the Elements, viz. iron, ruthenium, osmium, cobalt, rhodium, iridium, nickel, palladium or platinum,
W are preferably identical or different and are each a hydrogen atom, a C₁-C₂₀-hydrocarbon group, a halogen atom, an NR¹₂ group, where R¹ is a halogen atom, a C₁-C₁₀-alkyl group or a C₆-C₁₀-aryl group,
j is an integer which corresponds to the valence of the transition metal atom M²,
L is an uncharged donor ligand and i is 1, 2, 3, 4, 5 or 6.

3. A catalyst system as claimed in claim 2, wherein M¹ is a transition metal of group IVb of the Periodic Table of the Elements,
Y are preferably identical or different and are each a C₁-C₁₀-hydrocarbon group or a halogen atom and k is an integer which corresponds to the valence of the transition metal atom M¹ minus two, R⁷, R⁸, R⁹, R¹⁰, R¹¹, R¹², R¹³ and R¹⁴ are identical or different and are each a hydrogen atom, a C₁-C₁₀-hydrocarbon group or two or more adjacent radicals R⁷, R⁸, R⁹ or R¹⁰ or R¹¹, R¹², R¹³ or R¹⁴ together with the atoms connecting them form a ring system,
B is 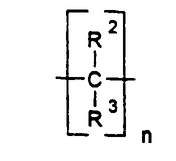 where n is an integer 1, 2, 3 or 4 and R² and R³ are identical and are each a hydrogen atom, a C₁-C₁₀-hydrocarbon group or R² and R³ together with the atoms connecting them form one or more rings,
D is
X-R⁵ ₂
where X is an element of group Va of the Periodic Table of the Elements, in particular nitrogen or phosphorus, and R⁵ are identical or different and are each a hydrogen atom, a C₁-C₁₀-hydrocarbon group or two radicals R⁵ together with the atoms connecting them form one or more rings,
M² is preferably a metal of group VIIIb of the Periodic Table of the Elements, particularly preferably ruthenium, cobalt, rhodium, nickel or palladium,
W are preferably identical and are each a hydrogen atom, a C₁-C₁₀-hydrocarbon group or a halogen atom, in particular bromine, chlorine or fluorine, or an NR¹₂ group, where R¹ is a halogen atom, a C₁-C₁₀-alkyl group or a C₆-C₁₀-aryl group, and j is an integer which corresponds to the valence of the transition metal atom M²,
L is an uncharged donor ligand and
i is 1, 2, 3 or 4.

4. A catalyst as claimed in one or more of claims 1 to 3, wherein the cocatalyst is an aluminum compound and/or a boron compound.

5. A process for preparing a polyolefin by polymerization of one or more olefins in the presence of the catalyst system as claimed in one or more of claims 1 to 4.

6. A process for the hydroformylation of an olefin with carbon monoxide and hydrogen in the presence of a bimetallic transition metal compound of the formula I as defined in claim 1.

7. A bimetallic transition compound of the formula I 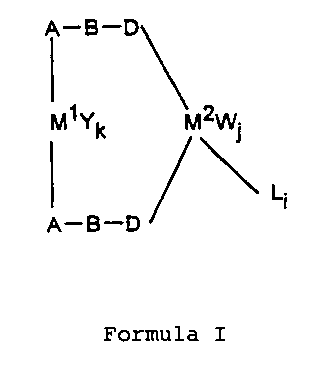 where
M¹ is a transition metal of group IIIb, IVb, Vb or VIb of the Periodic Table of the Elements,
Y is either identical or different and is each a hydrogen atom, a C₁-C₄₀-hydrocarbon group, an OH group, a halogen atom, an NR¹₂ group where R¹ is a halogen atom, a C₁-C₁₀-alkyl group or a C₆-C₁₀-aryl group, or
Y is a substituted or unsubstituted butadiene unit,
k is an integer which corresponds to the valence of the transition metal atom M¹ minus two and, when Y is butadiene, is equal to 1,
A are identical or different and are each a substituted or unsubstituted cyclopentadienyl ligand of which at least one has to be substituted,
B are identical or different and are 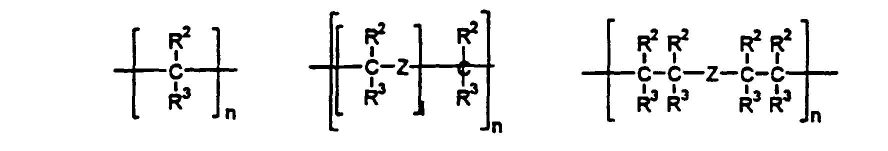 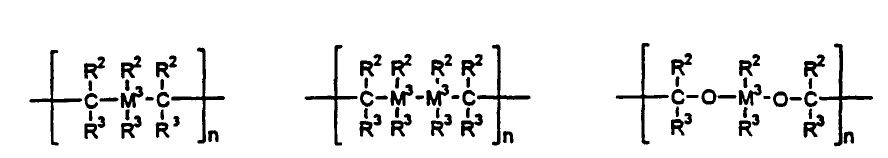 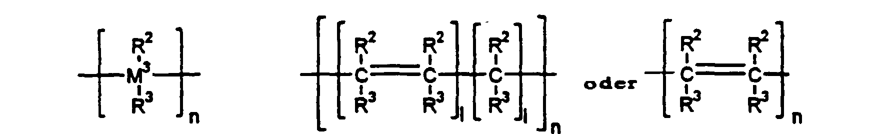 where n is an integer from 0 to 20, 1 is an integer from 1 to 20, Z is
〉NR⁴, 〉CO, 〉PR⁴, 〉P(O)R⁴, 〉SO,
SO₂, O or S, where R⁴ is a halogen atom, a C₁-C₁₀-alkyl group or a C₆-C₁₀-aryl group,
R² and R³ are identical or different and are each a hydrogen atom, a halogen atom, a C₁-C₄₀-hydrocarbon group such as a C₁-C₂₀-alkyl group, a C₁-C₂₀-alkoxy group, a C₆-C₂₀-aryl group, a C₂-C₂₀-alkenyl group, a C₇-C₄₀-alkylaryl group or a C₈-C₄₀-arylalkenyl group, or two radicals R², two radicals R³ or one radical R² and one radical R³, in each case together with the atoms connecting them, form one or more rings and M³ is silicon, germanium or tin,
D are identical or different and are
X-R⁵ ₘ,
and X is covalently bonded to B and coordinatively bonded to M², where X is nitrogen and R⁵ are identical or different and are each a hydrogen atom, a halogen atom, or a
C₁-C₄₀-hydrocarbon group, or two radicals R⁵ together with the atoms connecting them form one or more rings and m is 2, M² is a metal of group VIb, VIIb, VIIIb, IXb or Xb of the Periodic Table of the Elements,
W are either identical or different and are each a hydrogen atom, a C₁-C₄₀-hydrocarbon group, a halogen atom, an OR¹ group, an NR¹₂ group, where R¹ is a hydrogen atom, a halogen atom, a C₁-C₁₀-alkyl group or a C₆-C₁₀-aryl group, or W is a substituted or unsubstituted butadiene unit,
j is an integer 0, 1, 2, 3, 4, 5 or 6 which corresponds to the valence of the transition metal atom M²,
L is an uncharged donor ligand and
i is an integer from 0 to 10.

## Revendications

1. Système de catalyseur, contenant a) au moins un co-catalyseur et b) au moins un composé bimétallique de métal de transition de formule I 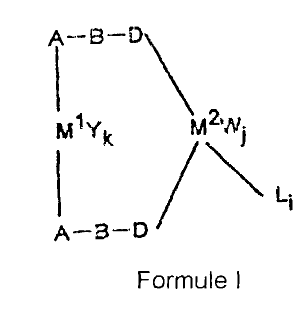 où
M¹ est un métal de transition du groupe IIIb, IVb, Vb ou Vlb de la classification périodique des éléments,
Y sont identiques ou différents et représentent un atome d'hydrogène, un groupement contenant un hydrocarbure en C₁-C₄₀, un groupement OH, un atome d'halogène, un groupement NR¹₂, où R¹ est un atome d'halogène, un groupement alkyle en C₁-C₁₀, ou un groupement aryle en C₆-C₁₀, ou bien
Y est un motif butadiène substitué ou non substitué,
k est un nombre entier, qui correspond à la valence de l'atome de métal de transition M¹ moins 2, et vaut 1 lorsque Y représente un butadiène,
A sont identiques ou différents et représentent un ligand cyclopentadiènyle substitué ou non substitué, l'un au moins d'entre eux devant être substitué,
B sont identiques ou différents et représentent 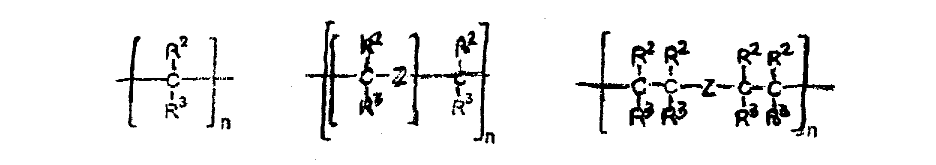 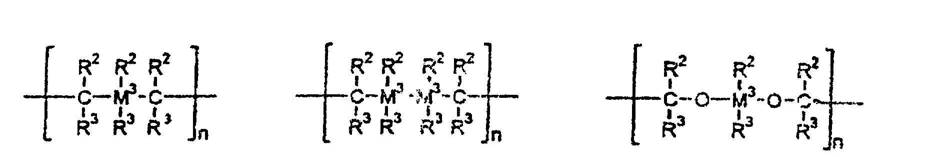 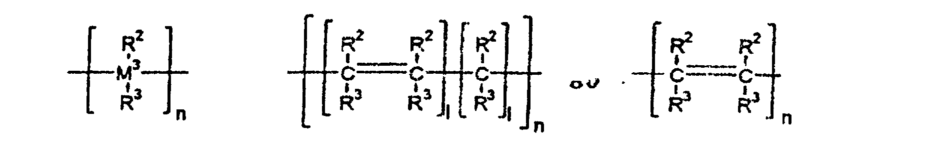 où n est un nombre entier de 0 à 20, l est un nombre entier de 1 à 20, Z représente
〉NR⁴, 〉CO, 〉PR⁴, 〉P(O)R⁴, 〉SO,
SO₂, O ou S, où R⁴ représente un atome d'halogène, un groupement alkyle en C₁-C₁₀ ou un groupement aryle en C₆-C₁₀,
R² et R³ sont identiques ou différents et représentent un atome d'hydrogène, un atome d'halogène, un groupement contenant un hydrocarbure en C₁-C₄₀, ou bien à chaque fois deux restes R², ou bien à chaque fois deux restes R³, ou bien encore un reste R² et un reste R³ forment un ou plusieurs cycles, avec les atomes qui les relient, et M³ est du silicium, du germanium ou de l'étain,
D sont identiques ou différents et représentent
X-R⁵ ₘ
et X est relié à B de façon covalente et à M² de façon coordinative, où X est un élément du groupe Va ou VIa de la classification périodique des éléments et R⁵ sont identiques ou différents et représentent un atome d'hydrogène, un atome d'halogène, un groupement contenant un hydrocarbure en C₁-C₄₀, ou bien à chaque fois deux restes R⁵ forment un ou plusieurs cycles avec les atomes qui les relient, et m vaut 1 ou 2, M² est un métal du groupe Vlb, Vllb, Vlllb, lXb ou Xb de la classification périodique des éléments,
W sont identiques ou différents et représentent un atome d'hydrogène, un groupement contenant un hydrocarbure en C₁-C₄₀, un atome d'halogène, un groupement OR¹, un groupement NR¹₂, où R¹ est un atome d'hydrogène, un atome d'halogène, un groupement alkyle en C₁-C₁₀ ou un groupement aryle en C₆-C₁₀, ou W représente un motif butadiène substitué ou non substitué,
j est un nombre entier 0, 1, 2, 3, 4, 5 ou 6, correspondant à la valence de l'atome de métal de transition M²,
L représente un ligand donneur neutre, et
i est un nombre entier de 0 à 10.

2. Système de catalyseur selon la revendication 1, où le composé bimétallique de métal de transition présente la formule (II) 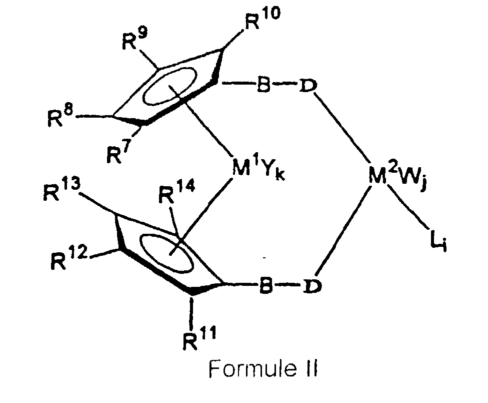 où
M¹ est un métal de transition du groupe IIIb, IVb, Vb ou Vlb de la classification périodique des éléments,
Y sont de préférence identiques ou différents et représentent un atome d'hydrogène, un groupement contenant un hydrocarbure en C₁-C₂₀, k est un nombre entier, qui correspond à la valence de l'atome de métal de transition M¹ moins 2,
R⁷, R⁸, R⁹, R¹⁰, R¹¹, R¹², R¹³ et R¹⁴ sont identiques ou différents et représentent un atome d'hydrogène, un atome d'halogène, un groupement contentant un hydrocarbure en C₁-C₄₀, un reste -SiR¹₃, -NR¹₂, -SiOR¹₃, -SiSR¹₃ ou -PR¹₂, où R¹ représente un atome d'halogène, un groupement alkyle en C₁-C₁₀ ou un groupement aryle en C₆-C₁₀, ou bien au moins deux restes voisins R⁷, R⁸, R⁹ et R¹⁰ ou R¹¹, R¹², R¹³ et R¹⁴ forment ensemble un système cyclique avec les atomes qui les relient, ou bien un reste R⁷, R⁸, R⁹ ou R¹⁰ représente avec un reste R¹¹, R¹², R¹³ ou R¹⁴ un pont entre les deux cycles cyclopentadiényle,
B représente 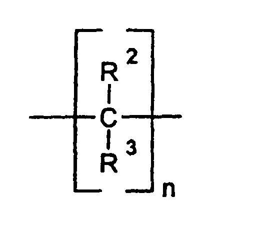 où n est un nombre entier de 1 à 8, en particulier 1, 2, 3 ou 4 et R² et R³ sont identiques ou différents et représentent un atome d'hydrogène, un atome d'halogène, un groupement contenant un hydrocarbure en C₁-C₂₀, ou bien à chaque fois deux restes R², ou bien à chaque fois deux restes R³, ou bien encore R² et R³ forment un ou plusieurs cycles, avec les atomes qui les relient,
D représente
X-R⁵ ₘ
où X est un élément du groupe Va de la classification périodique des éléments, tel que l'azote ou le phosphore, ou bien élément du groupe Vla de la classification périodique des éléments, tel que l'oxygène ou le soufre, et R⁵ sont identiques ou différents et représentent un atome d'hydrogène ou un groupement contenant un hydrocarbure en C₁-C₁₀, ou bien à chaque fois deux restes R⁵ forment un ou plusieurs cycles avec les atomes qui les relient,
et lorsque X est de l'azote ou du phosphore, m vaut 2, et lorsque X est de l'oxygène ou du soufre, m vaut 1,
M² est un métal du groupe Vlllb de la classification périodique des éléments, tel que le fer, le ruthénium, l'osmium, le cobalt, le rhodium, l'iridium, le nickel, le palladium ou le platine,
W sont identiques ou différents et représentent un atome d'hydrogène, un groupement contenant un hydrocarbure en C₁-C₂₀, un atome d'halogène, un groupement NR¹₂, où R¹ est un atome d'halogène, un groupement alkyle en C₁-C₁₀ ou un groupement aryle en C₆-C₁₀,
j est un nombre entier correspondant à la valence de l'atome de métal de transition M²,
L représente un ligand donneur neutre, et
i vaut 1, 2, 3, 4, 5 ou 6.

3. Système de catalyseur selon la revendication 2, où M¹ est un métal de transition du groupe IVb de la classification périodique des éléments, Y sont de préférence identiques ou différents et représentent un groupement contenant un hydrocarbure en C₁-C₁₀ ou un atome d'halogène et k est un nombre entier, qui correspond à la valence de l'atome de métal de transition M¹ moins 2,
R⁷, R⁸, R⁹, R¹⁰, R¹¹, R¹², R¹³ et R¹⁴ sont identiques ou différents et représentent un atome d'hydrogène, un groupement contentant un hydrocarbure en C₁-C₁₀, ou bien au moins deux restes voisins R⁷, R⁸, R⁹ ou R¹⁰ ou R¹¹, R¹², R¹³ ou R¹⁴ forment ensemble un cycle avec les atomes qui les relient,
B représente 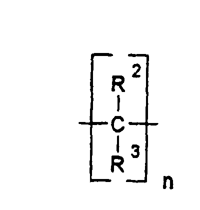 où n est un nombre entier 1, 2, 3 ou 4 et R² et R³ sont identiques et représentent un atome d'hydrogène, un groupement contenant un hydrocarbure en C₁-C₁₀, ou bien R² et R³ forment à chaque fois un ou plusieurs cycles, avec les atomes qui les relient,
D représente
X-R⁵ ₂
où X est un élément du groupe Va de la classification périodique des éléments, en particulier l'azote ou le phosphore, et R⁵ sont identiques ou différents et représentent un atome d'hydrogène, un groupement contenant un hydrocarbure en C₁-C₁₀, ou bien à chaque fois deux restes R⁵ forment un ou plusieurs cycles avec les atomes qui les relient, M² est de préférence un métal du groupe Vlllb de la classification périodique des éléments, en particulier de préférence le ruthénium, le cobalt, le rhodium, le nickel ou le palladium,
W sont de préférence identiques et représentent un atome d'hydrogène, un groupement contenant un hydrocarbure en C₁-C₁₀ ou un atome d'halogène, en particulier le brome, le chlore, le fluor ou un groupement NR¹₂, où R¹ est un atome d'halogène, un groupement alkyle en C₁-C₁₀ ou un groupement aryle en C₆-C₁₀, et
j est un nombre entier correspondant à la valence de l'atome de métal de transition M²,
L représente un ligand donneur neutre, et
i vaut 1, 2, 3 ou 4.

4. Catalyseur selon l'une quelconque des revendications 1 à 3, où le co-catalyseur est un composé de l'aluminium et/ou un composé du bore.

5. Procédé de préparation d'une polyoléfine par polymérisation d'une ou plusieurs oléfines en présence d'un système de catalyseurs selon l'une quelconque des revendications 1 à 4.

6. Procédé d'hydroformylation d'une oléfine avec du monoxyde de carbone et de l'hydrogène, en présence d'un composé bimétallique de métal de transition de formule I, défini comme dans la revendication 1.

7. Composé bimétallique de métal de transition de formule I 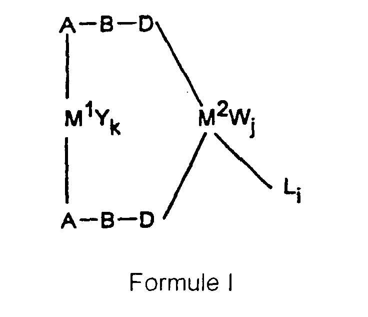 où M¹ est un métal de transition du groupe IIIb, IVb, Vb ou Vlb de la classification périodique des éléments,
Y sont identiques ou différents et représentent un atome d'hydrogène, un groupement contenant un hydrocarbure en C₁-C₄₀, un groupement OH, un atome d'halogène, un groupement NR¹₂, où R¹ est un atome d'halogène, un groupement alkyle en C₁-C₁₀, ou un groupement aryle en C₆-C₁₀, ou bien
Y est un motif butadiène substitué ou non substitué,
k est un nombre entier, qui correspond à la valence de l'atome de métal de transition M¹ moins 2, et vaut 1 lorsque Y représente un butadiène,
A sont identiques ou différents et représentent un ligand cyclopentadiènyle substitué ou non substitué, l'un au moins d'entre eux devant être substitué,
B sont identiques ou différents et représentent 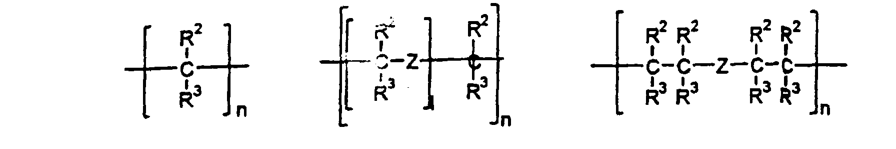 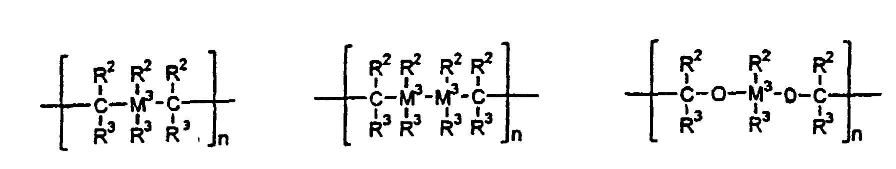 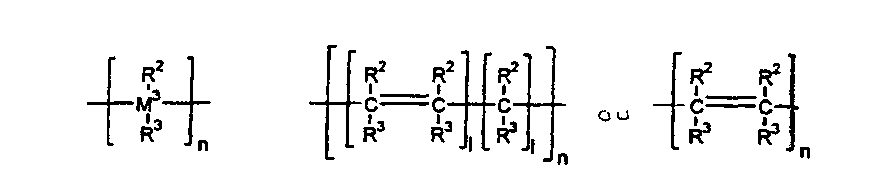 où n est un nombre entier de 0 à 20, l est un nombre entier de 1 à 20, Z représente
〉NR⁴, 〉CO, 〉PR⁴, 〉P(O)R⁴, 〉SO,
SO₂, O ou S, où R⁴ représente un atome d'halogène, un groupement alkyle en C₁-C₁₀ ou un groupement aryle en C₆-C₁₀,
R² et R³ sont identiques ou différents et représentent un atome d'hydrogène, un atome d'halogène, un groupement contenant un hydrocarbure en C₁-C₄₀ tel qu'un groupement alkyle en C₁-C₂₀, alcoxy en C₁-C₂₀, aryle en C₆-C₂₀, alcényle en C₂-C₂₀, alkylaryle en C₇-C₄₀ ou un groupement arylalcényle en C8-C40 ou bien à chaque fois deux restes R², ou bien à chaque fois deux restes R³, ou bien encore un reste R² et un reste R³ forment un ou plusieurs cycles avec les atomes qui les relient, et M³ est du silicium, du germanium ou de l'étain, D sont identiques ou différents et représentent
X-R⁵ ₘ
et X est relié à B de façon covalente et à M² de façon coordinative, où X est de l'azote et R⁵ sont identiques ou différents et représentent un atome d'hydrogène, un atome d'halogène ou un groupement contenant un hydrocarbure en C₁-C₄₀ ou bien à chaque fois deux restes R⁵ forment un ou plusieurs cycles avec les atomes qui les relient, et m vaut 2,
M² est un métal du groupe Vlb, Vllb, Vlllb, IXb ou Xb de la classification périodique des éléments,
W sont identiques ou différents et représentent un atome d'hydrogène, un groupement contenant un hydrocarbure en C₁-C₄₀, un atome d'halogène, un groupement OR¹, un groupement NR¹₂, où R¹ est un atome d'hydrogène, un atome d'halogène, un groupement alkyle en C₁-C₁₀ ou un groupement aryle en C₆-C₁₀, ou W représente un motif butadiène substitué ou non substitué,
j est un nombre entier 0, 1, 2, 3, 4, 5 ou 6, correspondant à la valence de l'atome de métal de transition M²,
L représente un ligand donneur neutre, et
i est un nombre entier de 0 à 10.
